# EUROPÄISCHE PATENTANMELDUNG

(11) **EP 4 563 067 A1**
(43) Veröffentlichungstag der Anmeldung: **04.06.2025**
(21) Anmeldenummer: 24215662.8
(22) Anmeldetag: 27.11.2024
(51) Int. Cl.: A61B 1/04, A61B 1/06, A61B 1/07

(54) **BELEUCHTUNGSVORRICHTUNG UND SYSTEM MIT EINER BELEUCHTUNGSVORRICHTUNG UND EINEM BILDGEBUNGSINSTRUMENT**

(30) Priorität: 28.11.2023 DE 102023133301
(71) Anmelder: Karl Storz SE & Co. KG, 78532 Tuttlingen (DE)
(72) Erfinder: Martin, Uwe, 78532 Tuttlingen (DE); Buschle, Lukas, 78532 Tuttlingen (DE); HUBER, Florian, 78532 Tuttlingen (DE); GÖBEL, Werner, 78532 Tuttlingen (DE)

(57) **Zusammenfassung**

Die vorliegende Erfindung betrifft eine Beleuchtungsvorrichtung (200) zur Bereitstellung von Beleuchtungslicht für ein Bildgebungsinstrument (202), insbesondere ein Endoskop (204) oder Exoskop (206), umfassend eine optische Schnittstelle (208) zur optischen Anbindung eines Bildgebungsinstruments (202) und
eine Beleuchtungseinheit (210), die dazu eingerichtet ist, Beleuchtungslicht an die optische Schnittstelle (208) zu liefern, umfassend ein erstes Leuchtelement (212), das dazu eingerichtet ist, Licht in einem ersten Spektralbereich (214) zu emittieren, ein zweites Leuchtelement (216), das dazu eingerichtet ist, Licht in einem zweiten Spektralbereich (218) zu emittieren, der bezüglich des ersten Spektralbereichs (214) langwelligeres Licht umfasst, und ein optisches Element (220), das vor dem zweiten Leuchtelement (216) angeordnet ist und das dazu eingerichtet ist, Licht zumindest eines Teilbereichs des ersten Spektralbereichs (214) abzuschwächen und Licht zumindest eines Teilbereichs des zweiten Spektralbereichs (218) durchzulassen.

Die vorliegende Erfindung betrifft zudem ein System (252) mit einer Beleuchtungsvorrichtung (200) und einem Bildgebungsinstrument (202).

## Beschreibung

Die vorliegende Erfindung betrifft eine Beleuchtungsvorrichtung und ein System mit einer Beleuchtungsvorrichtung und einem Bildgebungsinstrument.

Aus dem Stand der Technik sind Bildgebungsvorrichtungen wie beispielsweise endoskopische oder exoskopische Vorrichtungen bekannt, die Multispektral- oder Hyperspektralbilder erzeugen. Multispektral- oder Hyperspektralbilder weisen neben zwei räumlichen Dimensionen, wie sie etwa ein herkömmliches Bild einer Kamera hat, eine spektrale Dimension auf. Die spektrale Dimension umfasst mehrere Spektralbänder (Wellenlängenbänder). Multispektrale und hyperspektrale Bilder unterscheiden sich im Wesentlichen in der Anzahl an und der Breite von ihren spektralen Bändern. Zur Erzeugung der Multispektral- oder Hyperspektralbilder kann es notwendig sein, ein Untersuchungsgebiet mit einfarbigem Beleuchtungslicht zu beleuchten.

Es sind einige Bildgebungsvorrichtungen zur Erzeugung solcher Multispektral- oder Hyperspektralbilder, insbesondere im Kontext medizinischer Anwendungen, bekannt. In DE 20 2014 010 558 U1 ist beispielsweise eine Vorrichtung zur Aufnahme eines Hyperspektralbilds eines Untersuchungsgebietes eines Körpers beschrieben.

Wie in DE 10 2020 105 458 A1 beschrieben, eignen sich multispektrale und hyperspektrale Bildgebungsvorrichtungen insbesondere als endoskopische Bildgebungsvorrichtung. In dem Zusammenhang ist multispektrale und/oder hyperspektrale Bildgebung ein fundamentales Einsatzfeld beispielsweise zur Diagnostik sowie zur Beurteilung eines Erfolgs bzw. einer Qualität eines Eingriffs.

Daneben wird insbesondere im medizinischen Bildgebungsbereich Weißlichtbildgebung eingesetzt. Beobachtetes Gewebe wird mit Weißlicht beleuchtet, und mittels einer Kamera oder anderer Bilderfassungssensorik werden Bilder des Gewebes erzeugt, die dann einem Benutzer angezeigt werden können.

Ferner wird Fluoreszenzbildgebung eingesetzt, im Speziellen im medizinischen Bildgebungsbereich. Gewebe wird dabei planmäßig in einem bestimmten Wellenlängenbereich beleuchtet, um gezielt in bestimmte Entitäten, wie beispielsweise Gewebebereiche, eingebrachte fluoreszierende Farbstoffmoleküle anzuregen. Das daraufhin emittierte Licht mit größerer Wellenlänge kann durch einen geeignet gewählten Filter beobachtet werden, mittels dessen Anregungslicht ausgeblendet werden kann. Ferner kann mittels der Fluoreszenzbildgebung Autofluoreszenz von biologischem Gewebe untersucht werden.

Um ein breites Anwendungsgebiet mit einer Bildgebungsvorrichtung abzudecken, können multimodale Bildgebungsvorrichtungen vorgesehen werden. Multimodale Bildgebungsvorrichtungen gestatten es, wahlweise Weißlichtbilder und/oder Multispektralbilder und/oder Fluoreszenzbilder und/oder Hyperspektralbilder aufzunehmen. Beispiele für derartige Bildgebungsvorrichtungen sind multimodale Endoskope und multimodale Exoskope. Zur Realisierung unterschiedlicher Modi können Beleuchtungsvorrichtungen erforderlich sein, die in unterschiedlichen Beleuchtungsmodi betreibbar sind, um bedarfsweise Beleuchtungslicht in unterschiedlichen Spektralbereichen zu erzeugen. Dazu können die Beleuchtungsvorrichtungen zumindest zwei Leuchtelemente umfassen, die in unterschiedlichen Spektralbereichen Licht emittieren. Emittiertes Licht kann gemischt werden und/oder lediglich Licht eines der Leuchtelemente zur Beleuchtung des Untersuchungsgebiets vorgesehen werden.

Die Erfinder haben allerdings erkannt, dass sich die Leuchtelemente einer Beleuchtungsvorrichtung gegenseitig beeinflussen können. Beispielsweise kann ein Leuchtelement durch Elektrolumineszenz und/oder Phosphoreszenz Licht emittieren, wenn es von einem weiteren Leuchtelement angeleuchtet wird oder Streulicht auf das Leuchtelement trifft. Dies kann im Speziellen bei der Fluoreszenzbildgebung und/oder bei der Multi- und/oder Hyperspektralbildgebung zu einer erhöhten und ungewollten Hintergrundbeleuchtung oder zu Bildgebungsartefakten führen, wodurch eine Bildgebungsqualität beeinträchtigt werden kann, insbesondere da das auf diese Weise erzeugte zusätzliche und ungewollte Hintergrundlicht in einem unerwünschten Spektralbereich auftritt.

Ausgehend vom Stand der Technik liegt der Erfindung die Aufgabe zugrunde, Beleuchtungslicht zuverlässig und effizient bereitzustellen.

Diese Aufgabe wird erfindungsgemäß gelöst durch eine Beleuchtungsvorrichtung und ein System, wie sie hierin beschrieben und in den Ansprüchen definiert sind.

Die vorliegende Erfindung sieht vor, eine Beleuchtungsvorrichtung zur Bereitstellung von Beleuchtungslicht für ein Bildgebungsinstrument, insbesondere ein Endoskop oder Exoskop, bereitzustellen. Die Beleuchtungsvorrichtung umfasst eine optische Schnittstelle zur optischen Anbindung eines Bildgebungsinstruments und eine Beleuchtungseinheit, die dazu eingerichtet ist, Beleuchtungslicht an die optische Schnittstelle zu liefern. Die Beleuchtungseinheit umfasst ein erstes Leuchtelement, das dazu eingerichtet ist, Licht in einem ersten Spektralbereich zu emittieren, ein zweites Leuchtelement, das dazu eingerichtet ist, Licht in einem zweiten Spektralbereich zu emittieren, der bezüglich des ersten Spektralbereichs langwelligeres Licht umfasst, und ein optisches Element, das vor dem zweiten Leuchtelement angeordnet ist und das dazu eingerichtet ist, Licht zumindest eines Teilbereichs des ersten Spektralbereichs abzuschwächen und Licht zumindest eines Teilbereichs des zweiten Spektralbereichs durchzulassen.

Zudem sieht die vorliegende Erfindung vor, ein System, insbesondere ein optisches System, bereitzustellen. Das System umfasst eine erfindungsgemäße Beleuchtungsvorrichtung und ein Bildgebungsinstrument, insbesondere ein Endoskop oder Exoskop, das an die optische Schnittstelle der Beleuchtungsvorrichtung anbindbar und/oder angebunden ist.

Durch die erfindungsgemäße Beleuchtungsvorrichtung und das erfindungsgemäße System kann zuverlässig und effizient Beleuchtungslicht bereitgestellt werden. Durch diese Merkmale kann erreicht werden, dass eine Hintergrundbeleuchtung während eines Bildgebungsvorgangs besser kontrollierbar ist. Ungewollte oder unkontrollierte Hintergrundbeleuchtung, beispielsweise im falschen Spektralbereich, kann reduziert werden kann. Eine Bildgebungsqualität kann dadurch verbessert werden. Ein spektrales Profil des Beleuchtungslichts kann besser eingestellt werden. Im Speziellen in der Fluoreszenzbildgebung kann erreicht werden, dass ein Objektbereich mittels Beleuchtungslicht beleuchtet werden kann, das in kontrollierter Weise in einem gewünschten Spektralbereich emittiert wird und/oder ein besseres Signal-Rausch-Verhältnis aufweist. Die Erfinder haben erkannt, dass es vorteilhaft ist, das optische Element vor dem zweiten Leuchtelement anzuordnen, das Licht in dem langwelligeren Spektralbereich emittiert.

Ein Wellenlängenpeak kann grundsätzlich einen Bereich um ein lokales Maximum in einem Wellenlängenspektrum aufweisen. Die Breite des Bereichs kann etwa von den Spezifikationen eines bestimmen Leuchtelements abhängen. Links und rechts neben dem Wellenlängenpeak kann eine Lichtintensität und/oder relative Helligkeit größer als Null sein. Der Wellenlängenpeak kann eine Peakhöhe aufweisen, die durch das lokale Maximum definiert werden kann. Die Wellenlänge des Maximums kann beispielsweise als Emissionswellenlänge eines Leuchtelements angegeben werden. Es versteht sich, dass ein Emissionsspektrum eines Leuchtelements um die angegebene Wellenlänge den Bereich aufweisen kann. Der Wellenlängenpeak kann aus einem Hintergrundrauschen hervorragen und die Grenzen des Wellenlängenpeaks kann bei einer statistisch signifikanten Abweichung mit einem Signifikanzniveau von beispielsweise 5 % festgelegt sein. In anderen Worten weist der Wellenlängenpeak Grenzen bei jenen Wellenlängen auf, bei denen sich der Wellenlängenpeak deutlich von einem Hintergrundrauschen unterscheidet. Alternativ oder zusätzlich können die Grenzen des Wellenlängenpeaks bei Wellenlängen liegen, die 5 % und/oder 10 % der Peakhöhe entsprechen. Zwischen den Grenzen kann eine Lichtintensität und/oder relative Helligkeit eine Normalverteilung und/oder andere Verteilung aufweisen und/oder von einer unteren Grenze bis zu dem lokalen Maximum ansteigen und bis zu einer oberen Grenze abfallen.

In diesem Zusammenhang kann mit "Licht in einem Spektralbereich emittieren" gemeint sein, dass das emittierte Licht einen Wellenlängenpeak in dem Spektralbereich aufweist und/oder die Breite des Wellenlängenpeaks diesem Spektralbereich entspricht. Ferner kann darunter zu verstehen sein, dass ein entsprechendes Leuchtelement unter anderem Licht in dem Spektralbereich emittieren kann. Beispielsweise kann das Leuchtmittel in mehreren Spektralbereichen Licht emittieren, insbesondere Weißlicht. Ferner können auch mehrere Wellenlängenpeaks in dem entsprechenden Spektralbereich vorliegen. Grundsätzlich kann sich Licht in dem Spektralbereich von Licht in anderen Spektralbereichen durch eine signifikant und/oder deutlich höhere relative Helligkeit und/oder Intensität unterscheiden. Beispielsweise emittiert ein blaues Leuchtelement, insbesondere blaue LED, mit einer Emissionswellenlänge von 470 nm Licht mit einem Wellenlängenpeak in einem spezifischen Spektralbereich, z.B. ersten oder zweiten Spektralbereich, wobei der Wellenlängenpeak eine Peakhöhe bei einer Wellenlänge von 470 nm aufweist und der Wellenlängenpeak eine Breite von 50 nm aufweist. Der entsprechende Spektralbereich kann entsprechend von zumindest im Wesentlichen 445 nm bis zumindest im Wesentlichen 495 nm reichen.

Die Beleuchtungsvorrichtung kann ein eigenständiges Gerät umfassen, das separat handhabbar ist. Dazu kann die Beleuchtungsvorrichtung ein eigenes Gehäuse aufweisen, das zumindest im Wesentlichen die übrigen Komponenten der Beleuchtungsvorrichtung einhaust. Dadurch kann die Beleuchtungsvorrichtung beispielsweise auf einem Geräteturm in einem Operationssaal oder einem Behandlungszimmer bedarfsweise bereitstellbar sein. Die Beleuchtungsvorrichtung kann eine Art Modul ausbilden, das einem medizinischen System bedarfsweise hinzufügbar ist. Gemäß einigen Ausführungsformen kann die Beleuchtungsvorrichtung in eine Bildgebungsvorrichtung integriert sein und/oder Teil eines Bildgebungssystems sein. Eine Bildgebungsvorrichtung kann weitere Vorrichtungen zum Betrieb des Bildgebungsinstruments umfassen, beispielsweise eine Steuereinheit und/oder eine Recheneinheit. Das Bildgebungssystem kann etwa eine Anzeigevorrichtung umfassen, die dazu eingerichtet ist, eine Darstellung anhand von Bilddaten zu erzeugen, die mittels des Bildgebungsinstruments erstellt wurden.

Das Beleuchtungslicht kann für eine Beleuchtung eines Objektbereichs vorgesehen sein. Durch die Beleuchtung kann eine Abbildung des Objektbereichs mittels des Bildgebungsinstruments ermöglicht sein. Insbesondere wenn das Bildgebungsinstrument ein Endoskop und/oder ein Exoskop umfasst, kann Umgebungslicht nicht für eine Abbildung des Objektbereichs ausreichend sein. Das Bildgebungsinstrument kann in solchen Fällen eine Beleuchtungseinrichtung, umfassend etwa eine Linse, umfassen, mittels derer das Beleuchtungslicht auf den Objektbereich führbar ist. Gemäß einigen Ausführungsformen kann das Beleuchtungslicht Licht im sichtbaren Spektralbereich, insbesondere Weißlicht, umfassen, umfassend einen Spektralbereich von zumindest im Wesentlichen 380 nm bis 780 nm. Das Beleuchtungslicht kann bevorzugt Pseudoweißlicht umfassen, das mittels mehrerer einfarbiger Beleuchtungsmittel erzeugt wurde, deren emittiertes Licht gemischt und/oder kombiniert wurde. "Einfarbig" kann in diesem Zusammenhang einen schmalbandigen Spektralbereich von beispielsweise bis zu 100 nm, insbesondere bis zu 60 nm, bevorzugt bis zu 30 nm, Breite bedeuten.

Ferner kann das Beleuchtungslicht Licht in einem Spektralbereich unterhalb und/oder oberhalb des sichtbaren Lichts umfassen. Das Beleuchtungslicht kann Licht in einem Spektralbereich von beispielsweise 200 nm bis 1100 nm, insbesondere 250 nm bis 1000 nm, bevorzugt 300 nm bis 950 nm, umfassen. Das Beleuchtungslicht kann Infrarotlicht, insbesondere Nahinfrarotlicht, und/oder Ultraviolettlicht umfassen. Beispielsweise kann das Beleuchtungslicht zur Fluoreszenzbildgebung im Nahinfrarotbereich vorgesehen sein. Insbesondere kann das Beleuchtungslicht anwendungsspezifisch bereitstellbar sein. Das kann bedeuten, dass wahlweise Beleuchtungslicht mit einem breiten Spektralbereich oder einem schmalen Spektralbereich bereitstellbar sein kann. Insbesondere kann das Beleuchtungslicht mit einem schmalen Spektralbereich zur multi- und/oder hyperspektralen Bildgebung sowie zur Fluoreszenzbildgebung geeignet sein. "Schmal" kann in diesem Zusammenhang einen schmalbandigen Spektralbereich von beispielsweise bis zu 70 nm, insbesondere bis zu 40 nm, bevorzugt bis zu 20 nm, Breite bedeuten.

Grundsätzlich kann das Bildgebungsinstrument ein separat handhabbares Instrument, insbesondere medizinisches Instrument, umfassen. Das Bildgebungsinstrument kann ein eigenes Gehäuse und/oder einen Handgriff aufweisen. Mittels des Handgriffs kann das Bildgebungsinstruments handhabbar sein. Alternativ oder zusätzlich kann das Bildgebungsinstrument zur Kopplung an einen Roboter, insbesondere chirurgischen Roboter, vorgesehen sein.

Das Bildgebungsinstrument kann dazu eingerichtet sein, einen Objektbereich abzubilden und Bilddaten zu erzeugen. Dazu kann das Bildgebungsinstrument eine Bilderfassungsvorrichtung, insbesondere eine Kamera, umfassend zumindest einen Bildsensor, umfassen. Der zumindest eine Bildsensor kann in dem Spektralbereich des Beleuchtungslichts lichtempfindlich sein. Ferner kann der Bildsensor für langwelligeres Licht lichtempfindlich sein, als Beleuchtungslicht bereitstellbar ist. Das kann insbesondere zur Fluoreszenzbildgebung vorteilhaft sein. Das Bildgebungsinstrument kann beispielsweise zwei Bildsensoren umfassen, wobei einer der Bildsensoren im Spektralbereich sichtbaren Lichts lichtempfindlich ist und der andere Bildsensor im (Nah-)Infrarotbereich lichtempfindlich ist.

Zusätzlich kann das Bildgebungsinstrument zumindest einen, insbesondere bedarfsweise auswählbaren, Beobachtungsfilter aufweisen. Der Beobachtungsfilter kann bedarfsweise vor einem Bildsensor anordenbar sein und/oder permanent vor einem Bildsensor angeordnet sein. Der Beobachtungsfilter kann insbesondere einen Kantenfilter und/oder einen Bandpassfilter umfassen. Mittels des Beobachtungsfilters kann dadurch bedarfsweise Abbildungslicht, das von dem Objektbereich reflektiert und/oder emittiert wurde, in einem festgelegten Spektralbereich zu einem Bildsensor führbar sein. Wird etwa Fluoreszenzbildgebung durchgeführt, kann der festgelegte Spektralbereich einem Emissionsspektrum eines fluoreszierenden Farbstoffmoleküls im Objektbereich entsprechen und insbesondere ein Spektralbereich des Beleuchtungslicht zumindest abgeschwächt werden. Das fluoreszierende Farbstoffmolekül kann gezielt in ein Gewebe einbringbar sein, das mittels Fluoreszenzbildgebung untersucht werden soll.

Gemäß einigen Ausführungsformen kann zumindest einer der Bildsensoren und/oder die Bilderfassungseinrichtung zumindest teilweise außerhalb des Bildgebungsinstruments angeordnet sein. Beispielweise kann Abbildungslicht über eine Lichtschnittstelle aus dem Bildgebungsinstrument auskoppelbar sein und der Bilderfassungseinheit, insbesondere dem Bildsensor, zuführbar sein.

Zudem kann das Bildgebungsinstrument eine optische Schnittstelle zur Einkopplung von dem Beleuchtungslicht aufweisen. Mittels der optischen Schnittstelle kann das Bildgebungsinstrument an die Beleuchtungsvorrichtung angekoppelt werden und bereitgestelltes Licht in das Bildgebungsinstrument eingekoppelt werden. Das eingekoppelte Beleuchtungslicht kann entlang des Bildgebungsinstruments zu einer Beleuchtungseinrichtung des Bildgebungsinstruments führbar sein. Bevorzugt umfasst das Bildgebungsinstrument ein Endoskop und/oder ein Exoskop.

Ein Endoskop ist ein, insbesondere medizinisches, Bildgebungsinstrument, das zur Abbildung von einem Objektbereich innerhalb einer Kavität, insbesondere eines Patienten, eingerichtet ist. Um das Endoskop der Kavität zumindest teilweise zuzuführen, kann das Endoskop einen Schaft umfassen. Der Schaft kann dünn und länglich ausgebildet sein. Insbesondere kann der Schaft bis zu 2 m, bevorzugt bis zu 1 m, insbesondere bis zu 0,5 m lang sein und einen Durchmesser von beispielsweise bis zu 5 cm, insbesondere bis zu 3 cm, bevorzugt bis zu 1,5 cm aufweisen. An dem distalen Ende des Schafts kann eine Linse angeordnet sein, die dazu eingerichtet ist, Abbildungslicht von den Objektbereich zu sammeln. Gesammeltes Abbildungslicht kann entlang des Schafts zu einem proximalen Schaftbereich und/oder einem am proximalen Ende des Schafts angeordneten Handgriff führbar sein. Zur Führung des Abbildungslichts kann eine Stablinsenanordnung vorgesehen sein. Alternativ oder zusätzlich kann das Endoskop an seinem distalen Ende zumindest einen Bildsensor aufweisen, der dazu eingerichtet ist, Bilddaten zu erzeugen. In das Endoskop eingekoppeltes Beleuchtungslicht kann mittels einer Stablinsenanordnung und/oder eines Lichtleiters, insbesondere zumindest einer Glasfaser, entlang des Schafts von einem proximalen Abschnitt des Schafts zu einem distalen Abschnitt des Schafts führbar sein.

Gemäß einigen Ausführungsformen kann ein Endoskop eine Schnittstelle zur Ankopplung einer externen Kamera umfassen. Die Schnittstelle kann insbesondere eine optische Schnittstelle umfassen, zu der das Abbildungslicht lieferbar ist. Die externe Kamera kann unmittelbar an die Schnittstelle koppelbar sein und/oder mittels eines Lichtleiters ankoppelbar sein. Gemäß einigen Ausführungsformen kann die externe Kamera gemeinsam mit dem Bildgebungsinstrument wie ein einzelnes Instrument handhabbar sein.

Ein Exoskop ist ein, insbesondere medizinisches Bildgebungsinstrument, das zur Abbildung von einem oberflächlichen Objektbereich, insbesondere eines Patienten, eingerichtet ist. Das Exoskop muss zur Bildgebung keiner Kavität zugeführt werden, sondern kann extern von dem Patienten angeordnet werden.

Mittels einer optischen Schnittstelle kann eine Einkopplung und/oder Auskopplung von Licht erreichbar sein. Grundsätzlich kann mittels der optischen Schnittstelle Licht aus einer Vorrichtung auskoppelbar und in eine andere Vorrichtung einkoppelbar sein. Insbesondere kann das Ein- und Auskoppeln unter geringen Lichtverlusten durchführbar sein. Beispielsweise kann eine optische Schnittstelle einen Lichtwellenleiteranschluss umfassen. In dem Zusammenhang kann unter einer "optischen Anbindung" verstanden werden, dass das Beleuchtungslicht über die optische Schnittstelle dem Bildgebungsinstrument zuführbar ist. Die optische Schnittstelle kann zumindest eine Linse umfassen, die dazu eingerichtet ist, Beleuchtungslicht beleuchtungseinheitsseitig zu sammeln.

Die Beleuchtungseinheit kann dazu eingerichtet sein, das Beleuchtungslicht zu erzeugen. Insbesondere kann die Beleuchtungseinheit dazu eingerichtet sein, Beleuchtungslicht mit einem gewünschten spektralen Intensitätsprofil und/oder Helligkeitsprofil bereitzustellen. Das Intensitätsprofil kann bevorzugt einstellbar sein. Beispielsweise kann durch ein gezieltes Ansteuern des ersten und/oder des zweiten Leuchtelements das Intensitätsprofil einstellbar sein. Zudem kann die Beleuchtungseinheit einen Innenraum definieren. Das erste und/oder das zweite Leuchtelement kann dazu eingerichtet sein, Licht in den Innenraum zu emittieren. Insbesondere kann sich der Innenraum zwischen den Leuchtelementen erstrecken. Die Leuchtelemente können insbesondere an gegenüberliegenden Seiten des Innenraums angeordnet sein. Ferner können die Leuchtelemente an rechtwinklig zueinanderstehenden Seiten des Innenraums angeordnet sein. Die optische Schnittstelle kann an einer Seite des Innenraums angeordnet sein, die von den Seiten verschieden ist, an denen die Leuchtelemente angeordnet sind. Ferner kann die Beleuchtungseinheit zumindest eine optische Vorrichtung umfassen, mittels derer das emittierte Licht der Leuchtelemente kombinierbar und/oder mischbar ist. Alternativ oder zusätzlich kann das emittierte Licht mittels der optischen Vorrichtung zu der optischen Schnittstelle lieferbar sein. Die optische Vorrichtung kann zumindest einen dichroitischen Spiegel und/oder einen Strahlteiler umfassen.

Die Erfinder haben erkannt, dass beispielsweise durch Streulicht, durch Reflektion emittierten Lichts eines der Leuchtelemente an einer den Innenraum begrenzenden Oberfläche und/oder unmittelbar durch Beleuchtung mittels emittierten Lichts eines der Leuchtelemente das entsprechend andere der Leuchtelemente ungewollt beleuchtet werden kann. Alternativ oder zusätzlich kann Licht im ersten Spektralbereich durch die optische Vorrichtung hindurch auf das zweite Leuchtmittel fallen. Die Erfinder haben ferner erkannt, dass dadurch eine Bildgebungsqualität beeinträchtigt sein kann, weil das ungewollte angeleuchtete Leuchtelement auf das einfallende Licht reagiert.

Die Beleuchtungseinheit kann Leuchtelemente umfassen, deren Emissionsspektren gemeinsam zumindest einen Spektralbereich von 450 nm bis 850 nm abdecken.

Die Leuchtelemente können Licht in unterschiedlichen Spektralbereichen emittieren. Die unterschiedlichen Spektralbereiche können bereichsweise zumindest teilweise übereinstimmen.

Die Leuchtelemente können einfarbige und/oder schmalbandig emittierende LEDs (Leuchtdioden) und/oder Laserdioden umfassen. Ferner kann zumindest eines der Leuchtelemente eine Weißlicht-LED oder eine andere Weißlichtquelle oder zumindest ein breitbandig emittierendes Leuchtelement sein. Beispielsweise kann eines der Leuchtelemente, insbesondere das erste Leuchtelement, ein blaulichtemittierendes Leuchtelement umfassen. Ein weitere Leuchtelement, insbesondere das zweite Leuchtelement, kann ein weißlichtemittierendes Leuchtelement umfassen.

In einigen Ausführungsformen umfasst die Beleuchtungseinheit zumindest ein blaues Leuchtelement, zumindest ein rotes Leuchtelement, zumindest ein dunkelrotes Leuchtelement und zumindest ein Nah-IR-Leuchtelement (Nahinfrarot-Leuchtelement), insbesondere jeweils LEDs oder Laserdioden. Zusätzlich kann die Beleuchtungseinheit zumindest eine Weißlicht-LED oder eine andere Weißlichtquelle umfassen. Beispielsweise kann eine Peakhöhe eines Wellenlängenpeaks emittierten Lichts eines der Leuchtelemente bei 460 nm, bei 530 nm, bei 660 nm, bei 770 nm und/oder bei 940 nm liegen. Insbesondere kann die Peakhöhe des ersten Leuchtelements etwa bei 460 nm und die Peakhöhe des zweiten Leuchtelements bei 530 nm liegen. Gemäß anderen Ausführungsformen kann die Peakhöhe des ersten Leuchtelements etwa bei 770 nm und die Peakhöhe des zweiten Leuchtelements bei 940 nm liegen.

Es kann vorkommen, dass eine Komponente des zweiten Leuchtelements durch Licht in dem ersten Spektralbereich derart anregbar ist, dass das zweite Leuchtelement aufgrund der Anregung Licht im zweiten Spektralbereich emittiert. Beispielsweise kann die Komponente phosphoreszierende und/oder fluoreszierende Eigenschaften aufweisen und ein Absorptionsspektrum aufweisen, das zumindest teilweise dem ersten Spektralbereich entspricht. Durch Beleuchtung mit Licht im ersten Spektralbereich kann in der Folge eine Emission beispielsweise zumindest teilweise in dem zweiten Spektralbereich aufgrund der Phosphoreszenz auftreten. Es kann auch vorkommen, dass mittels Lichts im ersten Spektralbereich, das auf das zweite Leuchtelement trifft, Elektronen eines Halbleitermaterials des zweiten Leuchtelements anregbar sind, wodurch diese, wenn sie in einen Grundzustand zurückkehren, Licht emittieren. In einem solchen Fall kann die Komponente ein Halbleitermaterial umfassen. Beispielsweise kann das zweite Leuchtelement Galliumnitrid umfassen und Licht im grünen Spektralbereich emittieren. Das erste Leuchtelement kann Licht im blauen Spektralbereich emittieren. Auf das zweite Leuchtelement auftreffendes Licht, das mittels des ersten Leuchtelements emittiert wurde, kann Elektronen des zweiten Leuchtelements, insbesondere des Galliumnitrids, anregen und/oder dadurch Elektrolumineszenz des zweiten Leuchtelements hervorrufen.

Unter anderem durch diese beispielhaft aufgeführten Effekte kann das Beleuchtungslicht das emittierte Licht in dem zweiten Spektralbereich enthalten, obwohl das zweite Leuchtelement nicht gezielt zur Lichtemission angesteuert wurde. Das derart verunreinigte Beleuchtungslicht kann in der Folge auf den Objektbereich führbar sein, wodurch insbesondere bei der Durchführung einer Fluoreszenzbildgebung Bildgebungsartefakte entstehen können und/oder erhöhtes Hintergrundlicht auftreten kann. Beispielsweise kann bei der Fluoreszenzbildgebung Fluorescein als Farbstoff eingesetzt werden, dessen Emissionswellenlänge in dem zweiten Spektralbereich liegt. Reflektiertes Beleuchtungslicht in dem zweiten Spektralbereich, das aufgrund der Verunreinigung in dem Beleuchtungslicht enthalten ist, kann von dem Objektbereich reflektiert werden und fälschlicherweise als Fluoreszenzemission des Fluorescein-Farbstoffs gehalten werden. Grundsätzlich kann zudem das Beleuchtungslicht in dem zweiten Spektralbereich, das aufgrund der Verunreinigung in dem Beleuchtungslicht enthalten ist, zu einem erhöhtem Hintergrundlicht in einer Fluoreszenzbildgebungsabbildung führen, was die Bildqualität beeinträchtigt.

Erfindungsgemäß wird daher das optische Element vor dem zweiten Leuchtelement angeordnet. Das kann bedeuten, dass das optische Element in einer optischen Achse des zweiten Leuchtelements angeordnet wird. Die optische Achse kann insbesondere eine Normale einer ebenen Lichtemissionsfläche und/oder Oberfläche des betreffenden Leuchtelements umfassen. Die optische Achse kann sich auf die Hauptausbreitungsrichtung des von dem Leuchtelement emittierten Lichts beziehen. In der optischen Achse kann das Licht eine größte Intensität aufweisen und/oder in die Lichtemission am stärksten gebündelt sein. Ferner können die Leuchtelemente eine Linse umfassen und/oder jeweils eine Linse vor den Leuchtelementen angeordnet sein. Das optische Element kann vor der Linse angeordnet sein.

Das optische Element kann insbesondere derart vor dem zweiten Leuchtelement anordenbar sein, dass das optische Element zumindest im Wesentlichen eine Emissionsfläche des Leuchtelements abdeckt. Das optische Element kann beabstandet von dem Leuchtelement angeordnet sein. Beispielsweise kann der Abstand bis zu 4 cm, insbesondere bis zu 3 cm, bevorzugt bis zu 2 cm, besonders bevorzugt bis zu 1 cm betragen. Eine Fläche des optischen Elements kann größer sein als die Emissionsfläche des Leuchtelements. Gemäß einigen Ausführungsformen kann ein Schlitz zwischen dem optischen Element und dem Leuchtelement, der durch die Beabstandung auftreten kann, optisch abgedeckt sein. Das kann bedeuten, dass Licht zumindest im Wesentlich lediglich durch das optische Element hindurch auf das Leuchtelement auftreffen kann. Alternativ oder zusätzlich kann das optische Element derart dimensioniert sein, dass lediglich Licht in derart geringfügiger Intensität an dem optischen Element vorbei auf das Leuchtelement auftreten kann, dass eine durch dieses Licht hervorgerufene Emission die Bildgebungsqualität zumindest nicht im Wesentlichen beeinträchtigt. Grundsätzlich kann das bedeuten, dass Licht, insbesondere umfassend bezüglich des zweiten Spektralbereichs kurzwelligeres Licht, das optische Element zumindest im Wesentlichen durchtreten muss, um auf das zweite Leuchtelement zu fallen.

Insbesondere kann das optische Element dazu eingerichtet sein, Licht des ersten Spektralbereichs abzuschwächen und Licht des zweiten Spektralbereichs durchzulassen. Der Ausdruck "zumindest eines Teilbereichs" kann zumindest im Wesentlichen den gesamten ersten und/oder zweiten Spektralbereich betreffen.

Das optische Element kann eine zumindest teilweise lichtdurchlässige Scheibe umfassen, die aus einem Glas und/oder einem Kunststoff, insbesondere Plexiglas, gefertigt ist. Optische Eigenschaften der Scheibe können durch eine gezielte Auswahl des Glases und/oder Kunststoffs einstellbar sein. Alternativ oder zusätzlich kann das optische Element, insbesondere die Scheibe, zumindest eine optisch wirkende Beschichtung aufweisen und/oder es können optisch wirkende Folien und/oder dergleichen vor und/oder hinter der Scheibe angeordnet sein. Insbesondere kann das optische Element ein Filterbauteil und/oder einen einbaubaren Filter umfassen. Beispielsweise kann ein solcher Filter einen Rahmen aufweisen, der die Scheibe einfasst. Das optische Element kann in einem geeigneten Einbauschlitz und/oder dergleichen anordenbar, insbesondere in diesen einschiebbar, sein. Beispielsweise kann die Beleuchtungseinheit eine Filterhalterung umfassen, insbesondere umfassend den Einbauschlitz, die dazu eingerichtet ist, das optische Element zu halten. Das optische Element kann in der Filterhalterung fixierbar sein. Ferner kann das optische Element austauschbar mit einem anderen optischen Element sein, das beispielsweise andere optische Eigenschaften aufweist. Ein Benutzer kann beispielsweise das optische Element austauschen.

"Kurzwelliger" und/oder "langwelliger" kann eine Differenz von Peakhöhen von Wellenlängenpeaks von beispielsweise bis zu 100 nm, insbesondere bis zu 60 nm, bevorzugt bis zu 20 nm bedeuten. Insbesondere kann sich "Kurzwelliger" und/oder "langwelliger" auf eine Breite des Wellenlängenpeaks des ersten Spektralbereichs und/oder des zweiten Spektralbereichs beziehen. Beispielsweise kann der erste Spektralbereich, insbesondere der Wellenlängenpeak des ersten Spektralbereichs, den zweiten Spektralbereich, insbesondere den Wellenlängenpeak des zweiten Spektralbereichs, zumindest teilweise überlappen. Die Maxima der Wellenlängenpeaks können jedoch um bis zu 100 nm, insbesondere bis zu 60 nm, bevorzugt bis zu 20 nm, voneinander beabstandet sein.

Das optische Element kann dazu eingerichtet sein, Licht in dem ersten Spektralbereich zu absorbieren, zu beugen und/oder zu reflektieren, um es abschwächen. In einigen Ausführungsformen kann das optische Element dazu eingerichtet sein, Licht in dem ersten Spektralbereich zu blockieren. Das optische Element kann für Licht in dem ersten Spektralbereich zumindest im Wesentlichen undurchlässig sein. Reflektieren kann diffus Streuen umfassen. Unter Abschwächen kann verstanden werden, dass eine Intensität des Lichts in dem ersten Spektralbereich auf einen niedrigeren Wert reduziert wird. Insbesondere umfasst das Abschwächen eine Intensitätsverringerung um wenigstens 90 %, wenigsten 95 %, wenigstens 99 % oder sogar wenigstens 99,9 %. Die Abschwächung kann relativ zu der Eingangsintensität durchführbar sein. Das optische Element kann eine relative Transmission in unterschiedlichen Spektralbereich, insbesondere in dem ersten Spektralbereich und/oder dem zweiten Spektralbereich, aufweisen. Insbesondere ist Licht im ersten Spektralbereich mittels des optischen Elements derart abschwächbar, dass eine Bildgebungsqualität nicht beeinträchtigt ist. "Durchlässen" kann in dem Zusammenhang zumindest im Wesentlichen durchlassen bedeuten. Licht im zweiten Spektralbereich kann geringfügig abschwächbar sein, wobei mit "geringfügig" gemeint sein kann, dass eine Bildgebung zumindest im Wesentlichen nicht beeinflusst wird. Insbesondere kann das optische Element dazu eingerichtet sein, eine Intensität von Licht im zweiten Spektralbereich um höchstens 10 %, vorzugsweise um höchstens 5 % und besonders bevorzugt um zu verringern 1 % abzuschwächen.

Grundsätzlich kann die Beleuchtungseinheit und/oder insbesondere das erste Leuchtelement und/oder das zweite Leuchtelement einstellbar und/oder kalibierbar sein. Dadurch kann auf einen Einfluss des optischen Elements auf Lichtintensitäten reagiert werden und die Leuchtelemente gezielt derart ansteuerbar sein, dass der Einfluss im Bezug auf eine Bildgebung reduziert wird. Dadurch kann etwa auf eine Wirkung des optischen Elements reagiert werden und optimale Emissionsintensitäten der Leuchtelemente für einen Bildgebungsvorgang eingestellt werden.

Zudem kann das optische Element einen Kantenfilter umfassen, dessen optische Kante bei einer Kantenwellenlänge liegt und der unterhalb der Kantenwellenlänge abschwächend und oberhalb der Kantenwellenlänge durchlassend ausgebildet ist, wobei der erste Spektralbereich Licht mit Wellenlängen umfasst, die kleiner sind als die Kantenwellenlänge, und wobei der zweite Spektralbereich Licht mit Wellenlängen umfasst, die größer sind als die Kantenwellenlänge. Dadurch kann günstig und effizient gezielt Licht in dem ersten Spektralbereich abschwächbar sein. Der Kantenfilter kann einen optischen Hochpassfilter und/oder einen Bandpassfilter umfassen. Insbesondere kann unterhalb der Kantenwellenlänge eine Lichtdurchlässigkeit und/oder relative Transmission des Kantenfilters durch eine zumindest im Wesentlichen linear steigende Kurve beschreibbar sein. Die Steigung der Kurve kann anpassbar sein, insbesondere durch Auswahl eines Kantenfilters mit unterschiedlicher Ordnung, wobei eine höhere Ordnung eine größere Steigung der Kurve bedeuten kann. Die Kantenwellenlänge kann insbesondere derart wählbar sein, dass in dem ersten Spektralbereich eine relative Transmission beispielsweise bis zu 70 %, insbesondere bis zu 40 %, bevorzugt bis zu 20 %, beträgt. Die relative Transmission kann durch die Wahl eines größeren Abstands der Kantenwellenlänge zu der oberen Grenze des ersten Spektralbereichs reduzierbar sein.

Ferner kann das optische Element dazu eingerichtet sein, Licht des ersten Spektralbereichs zumindest im Wesentlichen vollständig zu blockieren. Dadurch kann das Beleuchtungslicht besonders geringe Verunreinigung aufweisen und/oder eine besonders effiziente und/oder bediensichere Beleuchtungsvorrichtung bereitstellbar sein. Eine Bildgebungsqualität kann verbessert werden. Vollständig blockieren kann eine Transmission des optischen Elements von bis zu 5 % bedeuten. In einigen Ausführungsformen kann das optische Element zumindest im Wesentlichen keine Transmission in dem ersten Spektralbereich aufweisen. Beispielsweise kann das optische Element einen Shutter umfassen, der dazu eingerichtet ist, kein einfallendes Licht durchzulassen und/oder das zweite Leuchtelement zumindest nahezu vollständig abzuschirmen.

Außerdem kann die Beleuchtungseinheit multimodal ausgebildet und in mehreren unterschiedlichen Beleuchtungsmodi betreibbar sein. Die Beleuchtungsmodi können zumindest einen Fluoreszenzmodus umfassen, in dem das erste Leuchtelement aktiviert ist und in dem das zweite Leuchtelement deaktiviert ist. Zudem können die Beleuchtungsmodi zumindest einen weiteren Beleuchtungsmodus umfassen, in dem zumindest das zweite Leuchtelement aktiviert ist. Vorteilhafterweise kann die Beleuchtungseinheit ein breites Anwendungsgebiet abdecken. Eine Beleuchtungseinheit kann für verschiedene Bildgebungsmethoden verwendbar sein. Dadurch können Kosten eingespart werden, indem weniger Geräte angeschafft, betrieben und gewartet werden müssen. Ferner kann während einer Behandlung zwischen verschiedenen Bildgebungsmodi geschaltet werden. Dadurch muss das Bildgebungsinstrument nicht mit einer anderen Beleuchtungsvorrichtung verbunden werden, um etwa von Weißlichtbildgebung zu Fluoreszenzbildgebung zu wechseln. Insbesondere können die Leuchtelemente wahlweise betreibbar sein und/oder ihre Lichtemission wahlweise einstellbar sein. Die Beleuchtungseinheit kann eine Steuereinheit umfassen, die dazu eingerichtet ist, die Leuchtelemente anzusteuern.

Die Erfinder haben erkannt, dass insbesondere in dem Fluoreszenzmodus das Vorsehen des optischen Elements wünschenswert sein kann, um eine hohe Qualität bei der Fluoreszenzbildgebung zu erreichen.

Dies liegt daran, dass zumindest eine Komponente des zweiten Leuchtelements durch Licht in dem ersten Spektralbereich derart anregbar sein kann, dass das zweite Leuchtelement aufgrund der Anregung Licht im zweiten Spektralbereich emittiert.

Das optische Element kann zudem dazu eingerichtet sein, Licht des ersten Spektralbereichs gemäß einem Anteil anteilig durchzulassen. Vorteilhafterweise kann ausgenutzt werden, dass das zweite Leuchtelement Licht in dem zweiten Spektralbereich durch Beleuchtung mittels Lichts des ersten Spektralbereichs emittiert. Eine Effizienz der Beleuchtungsvorrichtung kann verbessert werden. Zudem kann gezielt ein spektrales Profil des Beleuchtungslichts eingestellt werden.

Zudem kann der Anteil derart abgestimmt sein, dass in dem Fluoreszenzmodus von dem zweiten Leuchtelement emittiertes Licht, das auf eine Anregung durch Licht des ersten Leuchtelements zurückgeht, als Hintergrundlicht zur Darstellung anatomischer Strukturen verwendbar ist. Dadurch kann eine besonders benutzerfreundliche Durchführbarkeit der Fluoreszenzbildgebung erreicht werden und/oder weitere Information aus Fluoreszenzbildern gezogen werden. Vorteilhafterweise kann dazu ausgenutzt werden, dass das zweite Leuchtelement Licht in dem zweiten Spektralbereich durch Beleuchtung mittels Lichts des ersten Spektralbereichs emittiert. Eine Effizienz der Beleuchtungsvorrichtung kann weiter verbessert werden. Ohne optisches Element kann das Hintergrundlicht zu groß sein und eine Qualität der Fluoreszenzbildgebung beeinträchtigen. Das das optische Element kann das Hintergrundlicht jedoch derart einstellbar und/oder reduzierbar sein, dass es in vorteilhafterweise zu Bildgebung verwendbar ist.

Gemäß einigen Ausführungsformen kann das optische Element bewegbar gelagert sein und zwischen einer Abschwächungsposition, in der das optische Element in einer optischen Achse des ersten Leuchtelement angeordnet ist, und eine Freigabeposition, in der optische Element nicht in der optischen Achse des ersten Leuchtelements angeordnet ist, bewegbar sein. Das optische Element kann wahlweise eingesetzt werden und dadurch eine Flexibilität und/oder Funktionalität der Beleuchtungsvorrichtung verbessert werden. Beispielsweise kann ein Benutzer das optische Element bewegen.

Alternativ oder zusätzlich kann die Beleuchtungseinheit ferner einen Antrieb umfassen, der dazu eingerichtet ist, das optische Element zwischen der Abschwächungsposition und der Freigabeposition zu bewegen. Eine Benutzerfreundlichkeit kann verbessert werden. Der Antrieb kann einen Elektromotor umfassen. Ferner kann der Benutzer über eine Benutzeroberfläche ein Bewegen des optischen Elements veranlassen. Alternativ oder zusätzlich kann das Bewegen automatisiert erfolgen. Beispielsweise kann das optische Element automatisch in die Abschwächungsposition bewegbar sein, wenn die Beleuchtungsvorrichtung in dem Fluoreszenzmodus betrieben wird. Wenn die Beleuchtungsvorrichtung beispielsweise in einen Weißlichtmodus umgeschaltet wird, kann das optische Element automatisch in die Freigabeposition bewegbar sein. Ferner kann auch der Shutter automatisiert betrieben werden.

Außerdem kann eine Lichtdurchlässigkeit des optischen Elements schaltbar sein. Vorteilhafterweise kann eine Lichtdurchlässigkeit platzsparend realisierbar sein. Unter "schaltbar" kann insbesondere "elektrisch schaltbar" gemeint sein und unterscheidet sich von einem mechanischen Verschieben des optischen Elements. Beispielsweise kann das optische Element ein LCD-Fenster (Liquid Crystal Display) umfassen, dessen Lichtdurchlässigkeit sich ändert, wenn eine Spannung angelegt wird. Ferner kann das optische Element eine PDLC-Folie (Polymer Dispersed Liquid Crystal) umfassen, deren Lichtdurchlässigkeit reduziert wird, wenn eine Spannung angelegt wird. Ferner kann das optische Element ein elektrochromes Fenster umfassen, dessen Lichtdurchlässigkeit sich ändert, wenn eine Spannung angelegt wird.

Ferner kann die Beleuchtungsvorrichtung ein weiteres optisches Element umfassen, das vor dem ersten Leuchtelement angeordnet ist und das dazu eingerichtet ist, Licht des ersten Spektralbereichs durchzulassen und Licht abzuschwächen, das bezüglich des ersten Spektralbereichs langwelliger ist. Vorteilhafterweise können die Leuchtelemente optisch entkoppelt werden. Das kann bedeuten, dass sich die Leuchtelemente in Bezug auf eine Emission zumindest im Wesentlichen nicht beeinflussen. Alternativ oder zusätzlich kann eine gegenseitige Beeinflussung der Emission gezielt einstellbar sein, beispielsweise indem anteilig Licht des langwelligeren und/oder kurzwelligerem Spektralbereichs durchgelassen wird. Das weitere optische Element kann einen Tiefpassfilter umfassen und zumindest im Wesentlichen in gleicher Weise wie das optische Element vor dem zweiten Leuchtelement ausgebildet sein und/oder entsprechend anordenbar und/oder angeordnet sein.

Die Beleuchtungsvorrichtung kann ferner ein oder mehrere weitere/s Leuchtelement/e umfassen, das/die dazu eingerichtet ist/sind, in unterschiedlichen Spektralbereichen Licht zu emittieren. Zudem kann die Beleuchtungsvorrichtung ein oder mehrere weitere optische Elemente umfassen, wobei vor jedem der Leuchtelemente ein optisches Element angeordnet ist, das dazu eingerichtet ist, Licht des entsprechenden Leuchtelements durchzulassen und Licht abzuschwächen, das bezüglich des Lichts des entsprechenden Leuchtelements kurzwelliger ist. Vorteilhafterweise kann dadurch eine flexible Beleuchtungsvorrichtung bereitgestellt werden, die einen breiten Spektralbereich abdeckt. In einigen Ausführungsformen kann die Beleuchtungsvorrichtung fünf Leuchtelemente umfassen, die jeweils in einem unterschiedlichen Spektralbereich Licht emittieren. Die weiteren optischen Elemente können entsprechend dem optischen Element ausgebildet sein und/oder entsprechend anordenbar und/oder angeordnet sein. Die Leuchtelemente können beliebig wahlweise ansteuerbar sein. Es kann beispielsweise in dem Fluoreszenzmodus Licht lediglich eines der Leuchtelemente angesteuert werden. Dadurch kann eine Fluoreszenzbildgebung in einem breiten Spektralbereich möglich sein. Beispielsweise kann eines der Leuchtelemente dazu eingerichtet sein, Licht im nahinfraroten Spektralbereich zu emittieren. Zudem kann mittels der mehreren Leuchtelemente Pseudoweißlicht erzeugbar sein und/oder Licht mit einem über einen breiten Spektralbereich einstellbarem Intensitätsprofil erzeugbar sein.

Außerdem kann die Beleuchtungseinheit zumindest einen Strahlkombinierer umfassen, der dazu eingerichtet ist, Licht eines der Leuchtelement mit Licht eines anderen der Leuchtelemente zu kombinieren und in Richtung der optischen Schnittstelle zu führen. Die Beleuchtungseinheit kann dadurch eine kompakte Bauweise aufweisen und Lichtverluste können geringgehalten werden. Der Strahlkombinierer kann zumindest einen halbdurchlässigen Spiegel aufweisen, der dazu eingerichtet sind, Licht in einem Spektralbereich durchzulassen und Licht in dem Spektralbereich unterschiedlichen Spektralbereichen zu reflektieren und/oder absorbieren. Der Spiegel kann eine Beschichtung umfassen, aufgrund derer der Spiegel für Licht eines Wellenlängenbereiches reflektierend und für Licht zumindest eines anderen Wellenlängenbereichs transmittierend ist.

Außerdem kann die Beleuchtungseinheit zumindest einen gekreuzten Strahlkombinier umfassen, mittels dessen Licht von gegenüberliegenden Eingangsseiten zu einer Ausgangsseite ablenkbar ist, wobei auf den gegenüberliegenden Eingangsseiten des gekreuzten Strahlkombinierers jeweils zumindest eines der Leuchtelemente angeordnet ist. Eine kompakte und effiziente Beleuchtungseinheit kann dadurch bereitgestellt werden. Der gekreuzte Strahlkombinierer kann zumindest zwei der oben beschriebenen Spiegel aufweisen, die überkreuzt angeordnet sind und/oder sich insbesondere in einem zentralen Bereich schneiden. Ein solcher Strahlkombinierer kann vier Seiten aufweisen, von denen drei Seiten als Eingangsseiten und die vierte Seite als Ausgangsseite vorgesehen sein kann. Es können mehrere, insbesondere zwei, der Strahlkombinierer in Reihe hintereinander schaltbar sein. Der erste Strahlkombinierer kann drei Eingangsseiten aufweisen, von denen jede zu einem der Leuchtelemente ausgerichtet ist. Das emittierte Licht der Leuchtelemente kann zu der Ausgangsseite ablenkbar sein. In einer optischen Achse der Ausgangsseite kann eine Eingangsseite des zweiten Strahlkombinierers angeordnet sein. An den weiteren Eingangsseiten des zweiten Strahlkombinierers kann jeweils ein weiteres Leuchtelement angeordnet sein. Licht, das aus der Ausgangsseite des zweiten Strahlkombinierers austritt, kann zu der optischen Schnittstelle führbar sein. Dadurch kann emittiertes Licht von fünf Leuchtelementen kombinierbar sein und zu der optischen Schnittstelle führbar sein. Die Spiegel der Strahlkombinierer können an die entsprechend zugeordneten Leuchtelemente angepasst sind. Insbesondere können die Spiegel dazu eingerichtet sein, Licht in dem Spektralbereich des zugeordneten Leuchtelements zu reflektieren und außerhalb des Spektralbereichs zu transmittieren.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung einer Bildgebungsvorrichtung mit einer Beleuchtungsvorrichtung;
- Fig. 2: eine schematische Darstellung der Beleuchtungsvorrichtung;
- Fig. 3: schematische Transmissionskurven von Spiegeln der Beleuchtungsvorrichtung;
- Fig. 4: eine schematische perspektivische Darstellung einer weiteren Ausführungsform der Bildgebungsvorrichtung;
- Fig. 5: eine schematische Darstellung einer Beleuchtungsvorrichtung;
- Fig. 6: eine schematische Darstellung eines Lichtintensitätsdiagramms;
- Fig. 7: eine Halterung für ein optische Element in einer schematischen Darstellung;
- Fig. 8: die Halterung für das optische Element in einer anderen schematischen Darstellung;
- Fig. 9: eine schematische Darstellung eines bewegbar gelagerten optischen Elements;
- Fig. 10: eine schematische Darstellung eines Transmissionsdiagramms eines Kantenfilters; und
- Fig. 11: eine schematische Darstellung einer weiteren Ausführungsform eines optischen Elements.

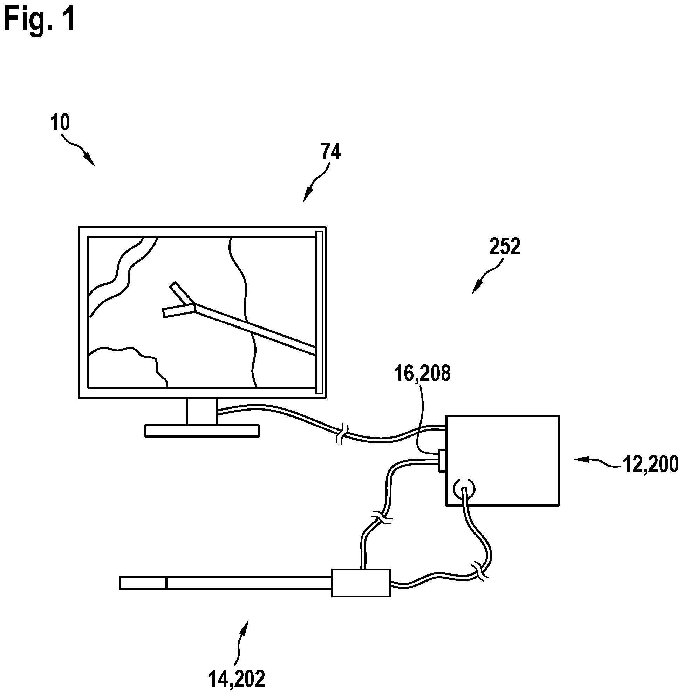

Mittels der Fig. 1 bis 4 wird eine Bildgebungsvorrichtung allgemein beschrieben. Fig. 1 zeigt eine schematische Darstellung einer Bildgebungsvorrichtung 10. Im exemplarisch dargestellten Fall ist die Bildgebungsvorrichtung 10 eine endoskopische Bildgebungsvorrichtung, konkret eine Endoskopvorrichtung. Alternativ könnte es sich bei der Bildgebungsvorrichtung 10 um eine exoskopische, eine mikroskopische oder eine makroskopische Bildgebungsvorrichtung handeln. Die Bildgebungsvorrichtung 10 ist beispielhaft als medizinische Bildgebungsvorrichtung gezeigt. Die Bildgebungsvorrichtung 10 ist beispielsweise zu einer Untersuchung einer Kavität vorgesehen.

Die Bildgebungsvorrichtung 10 weist ein medizinisches Bildgebungsinstrument 14 auf. Im dargestellten Fall handelt es sich hierbei um ein Endoskop 204.

Ferner umfasst die Bildgebungsvorrichtung 10 eine Beleuchtungsvorrichtung 12 mit einer optischen Schnittstelle 16. Das Bildgebungsinstrument 14 ist optisch an die optische Schnittstelle 16 anbindbar. Die optische Schnittstelle 16 kann Teil einer optisch-mechanischen Schnittstelle sein, die wahlweise lösbar und verbindbar ist. Das Bildgebungsinstrument 14 kann wahlweise von der Beleuchtungsvorrichtung 12 abkoppelbar sein. Die Beleuchtungsvorrichtung 12 ist dazu eingerichtet, Beleuchtungslicht an die optische Schnittstelle 16 zu liefern. Bei einer Bildgebung mittels des Bildgebungsinstrument 14 kann entsprechend die Beleuchtungsvorrichtung 12 das erforderliche Beleuchtungslicht bereitstellen, das zum Bildgebungsinstrument 14 geführt und von dort auf ein abzubildendes Objekt wie beispielsweise einen Situs ausgekoppelt wird.

Die Bildgebungsvorrichtung 10 umfasst im dargestellten Fall ferner eine Anzeigeeinheit, auf der Bilder angezeigt werden können, die auf Bilddaten beruhen, die mittels des Bildgebungsinstruments 14 erfasst wurden. Hierbei kann es sich um Videobilder, Standbilder, Überlagerungen unterschiedlicher Bilder, Teilbilder, Bildsequenzen etc. handeln.

Die Bildgebungsvorrichtung 10 ist multimodal. Exemplarisch ist die Bildgebungsvorrichtung in drei grundlegenden Modi betreibbar, einem Multispektralmodus, einem Fluoreszenzmodus und einem Weißlichtmodus. Ferner kann vorgesehen sein, dass die Bildgebungsvorrichtung 10 zusätzlich oder alternativ zum Multispektralmodus in einem Hyperspektralmodus betreibbar ist.

Die Beleuchtungsvorrichtung 12 ist multimodal. Die Beleuchtungsvorrichtung 12 ist in unterschiedlichen Beleuchtungsmodi betreibbar, in denen sie Licht für unterschiedliche Bildgebungsmodi liefert. Vorliegend ist die Beleuchtungsvorrichtung 12 in drei grundlegenden Modi betreibbar, einem Multispektralmodus, einem Fluoreszenzmodus und einem Weißlichtmodus. Ebenso ist das Bildgebungsinstrument 14 in unterschiedlichen Betriebsmodi betreibbar, konkret ebenfalls zumindest in einem Multispektralmodus, einem Fluoreszenzmodus und einem Weißlichtmodus. Im entsprechenden Betriebsmodus der Bildgebungsvorrichtung 10 werden die Modi der Beleuchtungsvorrichtung 12 und des Bildgebungsinstruments 14 aufeinander abgestimmt.

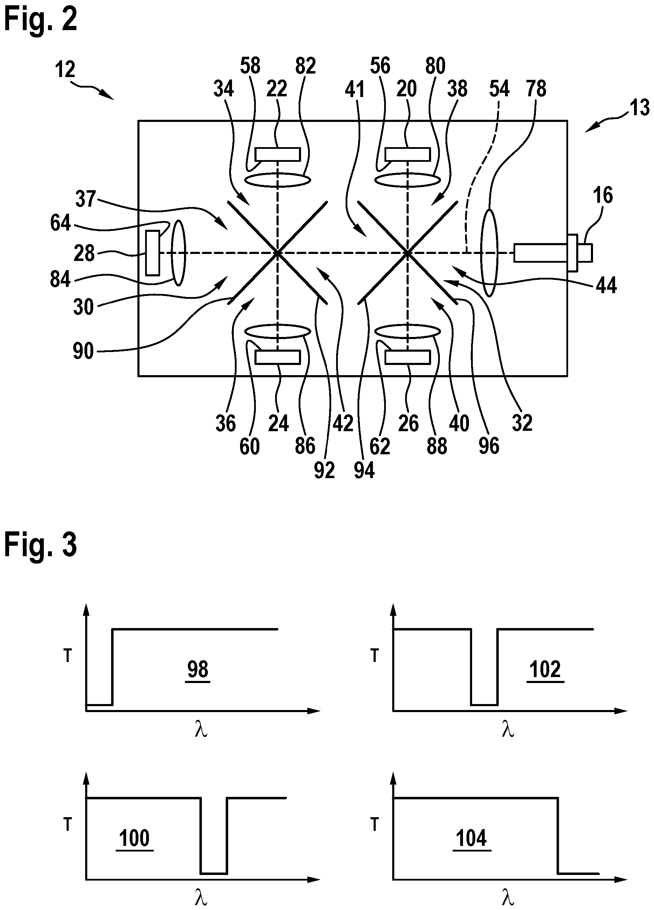

Fig. 2 zeigt eine schematische Darstellung der Beleuchtungsvorrichtung 12 umfassend eine Beleuchtungseinheit 13 und eine optische Schnittstelle 16. Die Beleuchtungsvorrichtung 12, insbesondere die Beleuchtungseinheit 13, umfasst mehrere unabhängig voneinander aktivierbare Leuchtelemente 20, 22, 24, 26, 28. Diese sind dazu eingerichtet, Licht gemäß unterschiedlichen Emissionsspektren zu emittieren, um Beleuchtungslicht zu liefern, d. h. das jeweilige Emissionsspektrum unterscheidet sich von Leuchtelement zu Leuchtelement.

Beispielhaft sind die Leuchtelemente 20, 22, 24, 26, 28 als LEDs ausgebildet. Konkret ist ein erstes Leuchtelement 20 als rote LED, ein zweites Leuchtelement 22 als dunkelrote LED, ein drittes Leuchtelement 24 als blaue LED und ein viertes Leuchtelement 26 als Nah-IR-LED ausgebildet. Die farbigen Leuchtelemente 20, 22, 24, 26 emittieren jeweils schmalbandig, beispielsweise mit Emissionspeak etwa bei den Wellenlängen 660 nm (erstes Leuchtelement 20), 770 nm (zweites Leuchtelement 22), 460 nm (drittes Leuchtelement 24) und 940 nm (viertes Leuchtelement 26).

Ferner ist ein fünftes Leuchtelement 28 vorgesehen, das vorliegend ein Weißlichtleuchtelement ist, etwa eine Weißlicht-LED. Das fünfte Leuchtelement 28 emittiert beispielsweise in einem Spektralbereich von etwa 400 bis 700 nm. In anderen Ausführungsformen können auch Laserdioden verwendet werden, insbesondere als farbige Leuchtelemente.

Alternativ kann als fünftes Leuchtelement 28 ein Leuchtelement vorgesehen sein, das Beleuchtungslicht in einem Teil des Spektralbereichs von 400 nm bis 700 nm, insbesondere in einem schmalbandigen Teil des Spektralbereichs, emittiert.

Je nach Beleuchtungsmodus werden einige der Leuchtelemente 20, 22, 24, 26, 28 zumindest zeitweise aktiviert, wohingegen ggf. andere Leuchtelemente 20, 22, 24, 26, 28 in dem betreffenden Beleuchtungsmodus nicht verwendet werden.

Vorliegend umfasst eine erste Gruppe das erste Leuchtelement 20 und das vierte Leuchtelement 26. Die erste Gruppe kann zusätzlich das Leuchtelement 22 und/oder das Leuchtelement 24 umfassen. Die erste Gruppe wird zur Multispektralbildgebung verwendet, wobei die enthaltenen Leuchtelemente 20, 26 sowie ggf. 22 und 24 jeweils als Stützstelle dienen. Im Multispektralmodus wird beispielsweise zunächst mit dem ersten Leuchtelement 20 beleuchtet und ein Bild aufgenommen. Anschließend wird mit dem vierten Leuchtelement 26 beleuchtet und ein Bild aufgenommen. Die Bilder beruhen jeweils auf Remission, d. h. es wird das vom abzubildenden Objekt zurückgestreute Licht betrachtet. Durch die beiden unterschiedlichen Stützstellen kann spektrale Information über das abzubildende Objekt gewonnen werden. Beispielsweise können hierdurch bestimmte Gewebearten, ein Perfusionszustand, eine Gewebebeschaffenheit oder dergleichen beurteilt werden.

Ferner umfasst eine zweite Gruppe das erste Leuchtelement 20, das zweite Leuchtelement 22 und das dritte Leuchtelement 24. Die zweite Gruppe wird zur Beleuchtung bei Fluoreszenzbildgebung verwendet. Hierbei können zum Beispiel gezielt mit geeignet gewählten Farbstoffen eingefärbte Objekte betrachtet werden. Auch können unterschiedliche Farbstoffe in unterschiedliche Gewebearten oder dergleichen eingebracht werden, die gleichzeitig betrachtet werden. Durch gezielte Anregung eines bestimmten Farbstoffs wird dieser zur Fluoreszenz angeregt. Abgebildet wird dann das Fluoreszenzlicht. Das erste Leuchtelement 20 ist beispielsweise dazu geeignet, den Farbstoff Cyanin 5.5 (Cy 5.5) anzuregen. Das zweite Leuchtelement 22 ist dazu geeignet, den Farbstoff Indocyaningrün (ICG) anzuregen. Das dritte Leuchtelement 24 ist dazu geeignet, den Farbstoff Fluoreszin anzuregen.

Des Weiteren umfasst eine dritte Gruppe das fünfte Leuchtelement 28. In der vorliegenden Ausführungsform umfasst die dritte Gruppe zudem das erste Leuchtelement 20 und das dritte Leuchtelement 24. Die dritte Gruppe dient dazu, Beleuchtungslicht für Weißlichtbildgebung bereitzustellen. Hierfür kann Weißlicht des fünften Leuchtelements 28 mit Licht bestimmter farbiger Leuchtelemente gemischt werden, wodurch spektrale Verluste ausgeglichen und/oder eine Farbtemperatur gezielt eingestellt werden kann.

Erkennbar sind einige der Leuchtelemente 20, 22, 24, 26, 28 mehreren Gruppen zugeordnet, beispielhaft das erste Leuchtelement 20 allen drei Gruppen sowie das dritte Leuchtelement 24 und ggf. auch das zweite Leuchtelement 22 der zweiten und der dritten Gruppe.

Alternativ oder zusätzlich kann auch vorgesehen sein, dass einige oder sämtliche der Leuchtelement 20, 22, 24, 26, 28 in einem Hyperspektralmodus eingesetzt werden. Es wird dann ein breites Anregungsspektrum erzeugt. In Kombination mit einem geeigneten Hyperspektraldetektor kann dann über das gesamte sichtbare und Nah-IR-Spektrum spektrale Information bzgl. des abzubildenden Objekt erfasst werden. Das Bildgebungsinstrument 14 kann zu diesem Zweck eine Pushbroom-Anordnung als Hyperspektraldetektor umfassen. In anderen Ausführungsformen wird eine Whiskbroom-Anordnung, eine Staring-Anordnung und/oder eine Schnappschuss-Anordnung verwendet. Das Bildgebungsinstrument 14 kann ein hyperspektrales Bildgebungsinstrument sein. Bezüglich unterschiedlicher Methoden einer hyperspektralen Bildgebung sowie hierfür erforderlicher Komponenten wird auf den Fachartikel "Review of spectral imaging technology in biomedical engineering: achievements and challenges" von Quingli Li et al. Erschienen in Journal of Biomedical Optics 18(10), 100901, Oktober 2013, sowie auf den Fachartikel "Medical hyperspectral imaging: a review" von Guolan Lu und Baowei Fei, erschienen in Journal of Biomedical Optics 19(1), 010901, Januar 2014, verwiesen.

Die Beleuchtungsvorrichtung 12 umfasst zwei Strahlkombinierer 30, 32. In dem Zusammenhang mit den Figuren 1 bis 4 wird die Funktion der Strahlkombinierer 30, 32 allgemein beschrieben. Die Strahlkombinierer 30, 32 umfassen jeweils eine Ausgangsseite 42, 44, jeweils eine der Ausgangsseite 42, 44 gegenüberliegende Eingangsseite 37, 41 und jeweils zwei einander gegenüberliegende Eingangsseiten 34, 36, 38, 40. Sämtliche Eingangsseiten 34, 36, 37, 38, 40, 41 führen einfallendes Beleuchtungslicht zumindest teilweise zur entsprechenden Ausgangsseite 42, 44. Die Ausgangsseite 42 eines ersten Strahlkombinierers 30 ist eine Eingangsseite 41 des zweiten Strahlkombinierers 32 zugewandt. Die Ausgangsseite 44 des zweiten Strahlkombinierers 32 ist der optischen Schnittstelle 16 zugewandt. Die beiden Strahlkombinierer 30, 32 sind vorzugsweise zueinander und/oder zur optischen Schnittstelle koaxial angeordnet.

Die Beleuchtungsvorrichtung 12 kann geeignete optische Elemente wie Linsen und/oder nicht dargestellte Spiegel umfassen. Exemplarisch sind in Fig. 2 mehrere Linsen 78, 80, 82, 84, 86, 88 dargestellt. Eine Linse 78 ist etwa der optischen Schnittstelle 16 zugeordnet und koppelt von der Ausgangsseite 44 des zweiten Strahlkombinierers 32 kommendes Licht in die optische Schnittstelle 16 ein. Ferner kann jedem der Leuchtelemente 20, 22, 24, 26, 28 jeweils eine Linse 80, 82, 84, 86, 88 zugeordnet sein. Ein besonders hoher Grad an Kompaktheit kann insbesondere dann erzielt werden, wenn die Leuchtelemente 20, 22, 24, 26, 28 jeweils ohne zwischengeordneten Spiegel an Eingangsseiten 34, 36, 37, 38, 40 des zumindest einen Strahlkombinierers 30, 32 angeordnet sind. Die Leuchtelemente 20, 22, 24, 26, 28 können dann sehr nah an den zumindest einen gekreuzten Strahlteiler 30, 32 herangerückt werden.

Die Strahlkombinierers 30, 32 umfassen jeweils zwei Spiegel 90, 92, 94, 96. Diese können grundsätzlich teildurchlässig sein, sodass Beleuchtungslicht von allen Eingangsseiten 34, 36, 37, 38, 40, 41 zur jeweiligen Ausgangsseite 42, 44 umgelenkt wird. In der vorliegenden Ausführungsform sind die Spiegel 90, 92, 94, 96 selektiv lichtdurchlässig. Dies ist mit weiterer Bezugnahme auf Fig. 3 veranschaulicht. Die Spiegel 90, 92, 94, 96 können Filter sein, die lediglich in einem definierten Bereich reflektieren, ansonsten aber eine hohe Transmission aufweisen. In Fig. 3 sind Transmissionskurven 98, 100, 102, 104 der Spiegel 90, 92, 94, 96 der beiden Strahlkombinierer 30, 32 dargestellt. Jedem der farbigen Leuchtelemente 20, 22, 24, 26 bzw. jeder der gegenüberliegenden Eingangsseiten 34, 36, 38, 40 ist einer der Spiegel 90, 92, 94, 96 zugeordnet. Die Spiegel 90, 92, 94, 96 sind dabei derart gewählt, dass diese jeweils in demjenigen Wellenlängenbereich reflektieren, in dem das zugeordnete Leuchtelement 20, 22, 24, 26 emittiert, daneben aber weitgehend transmittieren. Hierfür können im mittleren Wellenlängenbereich Kerbfilter verwendet werden, die beispielhaft die Transmissionsspektren 100 und 102 aufweisen können. An spektralen Rändern können anstelle von Kerbfiltern auch Hochpass- oder Tiefpass-Filter verwendet werden, vgl. Transmissionsspektren 98 und 104.

Aufgrund der spezifischen Transmissionsspektren 98, 100, 102, 104 der Strahlkombinierer 30, 32 wird Beleuchtungslicht des fünften Leuchtelements 28 spektral beschnitten. Es kann daher zweckmäßig sein, das durch die Strahlkombinierer 30, 32 geblockte Beleuchtungslicht gezielt mittels der Leuchtelemente 20 und 24, ggf. auch 22 und/oder 26 zu ergänzen. Hierdurch kann Beleuchtungslicht speziell in denjenigen Spektralbereichen ergänzt werden, in denen die Strahlkombinierer 30, 32 Beleuchtungslicht des fünften Leuchtelements 28 absorbieren und/oder reflektieren, jedenfalls aber nicht zur optischen Schnittstelle 16 transmittieren. Die ergänzend eingesetzten Leuchtelemente 20, 24 und ggf. 22 werden dabei vorzugsweise mit verringerter Leistung bzw. mit angepasster Leistung betrieben. Hierbei kann darauf abgezielt werden, das ursprüngliche Spektrum des fünften Leuchtelements 28 zumindest weitgehend wiederherzustellen.

In einigen Ausführungsformen kann das fünfte Leuchtelement 28 alternativ ein grünes Leuchtelement sein, bzw. allgemein ausgedrückt ein farbiges Leuchtelement, das vorrangig in demjenigen Spektralbereich emittiert, den der zumindest eine Strahlkombinierer30, 32 transmittiert. Beispielsweise kann das fünfte Leuchtelement 26 in solchen Ausführungsformen eine LED mit einem Emissionspeak bei etwa 530 nm sein. Infrage kommt hierfür auch eine grüne Laserdiode. Dabei kann vorgesehen sein, dass im Weißlichtmodus eine Farbmischung erfolgt und insbesondere keine individuelle Weißlichtquelle wie eine Weißlicht-LED zum Einsatz kommt, sondern Weißlicht aus separaten Leuchtelementen gezielt gemischt wird.

Es versteht sich, dass im Fall geeigneter Farbstoffe ein solches grünes Leuchtelement ebenfalls im Fluoreszenzmodus verwendbar sein kann. Alternativ oder zusätzlich könnte es im Multispektralmodus verwendbar sein.

Die Beleuchtungsvorrichtung 12 definiert einen gemeinsamen optischen Pfad 54, in den emittiertes Licht der Leuchtelemente 20, 22, 24, 26, 28 einkoppelbar ist. Der gemeinsame optische Pfad 54 erstreckt sich ausgehend von der Ausgangsseite 44 des zweiten Strahlkombinierers 32 zur optischen Schnittstelle 16. Der gemeinsame optische Pfad 54 ist vorliegend koaxial mit dem fünften Leuchtelement 26 angeordnet.

In der gezeigten Ausführungsform sind die Leuchtelemente 20, 26 der ersten Gruppe derart angeordnet, dass von den Leuchtelementen 20, 26 emittiertes Beleuchtungslicht ausgehend vom jeweiligen Leuchtelement 20, 26 bis zur optischen Schnittstelle 16 jeweils einen zumindest im Wesentlichen gleich langen Lichtweg durchläuft. Die Leuchtelemente 20, 26 der ersten Gruppe weisen jeweils eine lichtemittierende Fläche 56, 58 auf. Die lichtemittierenden Flächen 56, 62 sind bezüglich des gemeinsamen optischen Pfads 54 äquidistant angeordnet. Dies ist vorliegend dadurch erreicht, dass die beiden Leuchtelemente 20, 26 im gleichen Abstand von dem ihnen zugeordneten Strahlkombinierer 32 (vorliegend exemplarisch der zweite Strahlkombinierer 32), im Speziellen von dessen gegenüberliegenden Eingangsseiten 38, 40, angeordnet sind. Das Licht wird dabei vom Strahlkombinierer 32 in den gemeinsamen optischen Pfad 54 eingekoppelt.

Die Strahlkombinierer30, 32 sind insbesondere derart angeordnet, dass lichtemittierende Flächen 56, 58, 60, 62, 64 der Leuchtelemente 20, 22, 24, 26, 28 jeweils bezüglich ihres zugeordneten Strahlkombinierers 30, 32 äquidistant angeordnet sind.

Durch die Verwendung Strahlkombinierer 30, 32 und für unterschiedliche Modi gemeinsam verwendbarer Leuchtelemente 20, 22, 24, 26, 28 weist die Beleuchtungsvorrichtung 12 einen hohen Grad an Kompaktheit auf. Zudem kann durch die äquidistante Anordnung erreicht werden, dass keine spektralen Verschiebungen auftreten, wenn das Bildgebungsgerät 14 bzw. dessen Lichtleiter relativ zu der optischen Schnittstelle 16 verdreht wird.

Es versteht sich, dass eine andere Anzahl von Leuchtelementen 20, 22, 24, 26, 28 und/oder eine andere Anzahl Strahlkombinierer 30, 32 verwendet werden kann.

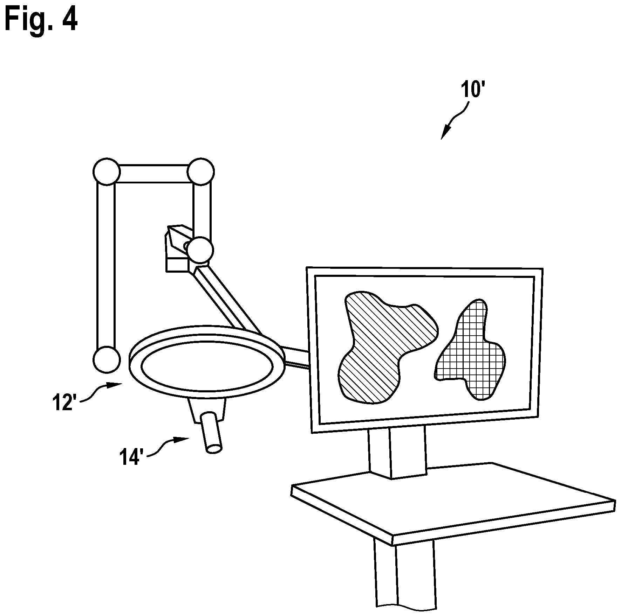

Fig. 4 zeigt eine schematische perspektivische Darstellung einer weiteren Ausführungsform einer Bildgebungsvorrichtung 10`. Die Bezugszeichen dieser Ausführungsform sind zur Unterscheidung mit Hochkommata versehen. Die Bildgebungsvorrichtung 10` ist in dieser Ausführungsform als exoskopische Bildgebungsvorrichtung ausgebildet. Sie umfasst eine Beleuchtungsvorrichtung 12' und ein Bildgebungsgerät 14`. Deren grundlegende Funktionsweise entspricht der oben beschriebenen, allerdings ist das Bildgebungsgerät 14' in dieser Ausführungsform als Exoskop (206) ausgebildet.

Die Erfinder haben erkannt, dass bei einer Anordnung gemäß der Fig. 2 eines der Leuchtelemente 20, 22, 24, 26, 28 eine Komponente umfassen kann, die durch Licht eines anderen der Leuchtelemente 20, 22, 24, 26, 28 zur Emission von Licht angeregt werden kann.

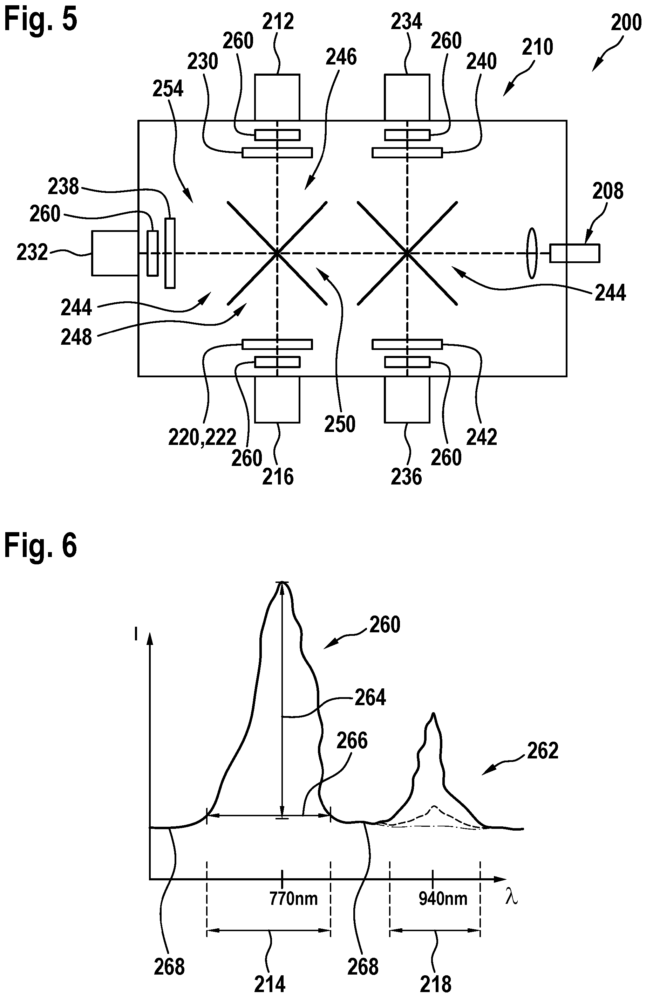

Die Fig. 5 zeigt eine erfindungsgemäße Beleuchtungsvorrichtung 200 in einer schematischen Ansicht. Die erfindungsgemäße Beleuchtungsvorrichtung 200 kann gemeinsam mit der Bildgebungsvorrichtung 10 betrieben werden. Das Bildgebungsinstrument 14 kann in diesem Fall das Bildgebungsinstrument 202 darstellen und die Fig. 1 das System 252 zeigen. Das System 252 umfasst eine Beleuchtungsvorrichtung 200, die anstelle der Beleuchtungsvorrichtung 12 vorsehbar sein kann. Das Bildgebungsinstrument 202 kann an die optische Schnittstelle 208 der Beleuchtungsvorrichtung 200 anbindbar sein und/oder ist an diese angebunden.

Ferner weisen die Fig. 2 und die Fig. 5 große Gemeinsamkeiten auf. Es wird daher vorwiegend auf Unterschiede eingegangen. Die Beleuchtungsvorrichtung 200 definiert einen Innenraum 254, in den Leuchtelemente 212, 216, 232, 234, 236 Licht hineinemittieren. Die Leuchtelemente 212, 216, 232, 234, 236 sind an drei Seiten des Innenraums angeordnet. An einer vierten Seite ist die optische Schnittstelle 208 angeordnet. Ein erstes Leuchtelement 212 ist auf einer gegenüberliegenden Seite eines zweiten Leuchtelements 216 angeordnet. Äquidistant von beiden Leuchtelementen 212, 216 ist ein erster Strahlkombinierer 244 innerhalb des Innenraums 254 angeordnet. Die Strahlkombinierer 244 der Fig. 2 können zumindest im Wesentlichen den Strahlkombinierern 30, 32 entsprechen. Der Strahlkombinierer 244 weist eine Eingangsseite 246 auf, die in einer optischen Achse des ersten Leuchtelements 212 angeordnet ist und die sich in Richtung des ersten Leuchtelements 212 öffnet. Eine zweite Eingangsseite 248 ist in einer optischen Achse des zweiten Leuchtelements 216 angeordnet und öffnet sich in Richtung des zweiten Leuchtelements 216. Die optischen Achsen der Leuchtelemente 212, 216 sind konzentrisch angeordnet. Mittels des Strahlkombinierers 244 wird in bereits beschriebener Weise Licht in Richtung der Ausgangsseite 250 umgelenkt, wobei emittiertes Licht der Leuchtelemente 212, 216 kombiniert werden kann. Ferner kann das Licht zu der optischen Schnittstelle 208 geführt werden. Alle Leuchtelemente 212, 216, 232, 234, 236 können gezielt angesteuert werden und/oder einzeln aktiviert werden, beispielsweise mittels einer Steuereinheit (nicht dargestellt). Die Beleuchtungsvorrichtung 200 ist multimodal ausgebildet und in mehreren unterschiedlichen Beleuchtungsmodi betreibbar. Die Beleuchtungsmodi umfassen einen Fluoreszenzmodus, in dem das erste Leuchtelement 212 aktiviert ist und in dem das zweite Leuchtelement 216 deaktiviert ist. In einem weiteren Beleuchtungsmodus ist zumindest das zweite Leuchtelement 216 aktiviert.

Das erste Leuchtelement 212 ist dazu eingerichtet, Licht in einem ersten Spektralbereich zu emittieren. Vorliegend ist das erste Leuchtelement ein blaues Leuchtelement, insbesondere blaue LED, mit einer Emissionswellenlänge von 470 nm. Das zweite Leuchtelement 216 ist dazu eingerichtet, Licht in einem zweiten Spektralbereich zu emittieren, der bezüglich des ersten Spektralbereichs langwelligeres Licht umfasst. Das zweite Leuchtelement 216 ist gemäß Fig. 5 ein grünes Leuchtelement, insbesondere grüne LED, mit einer Emissionswellenlänge von 530 nm. Das zweite Leuchtelement 216 umfasst eine Komponente, die durch Licht in dem ersten Spektralbereich derart anregbar ist, dass das zweite Leuchtelement aufgrund der Anregung Licht im zweiten Spektralbereich emittiert. Um diese Emission zumindest teilweise zu verhindern, ist das optische Element 220 vor dem zweiten Leuchtelement 216 angeordnet. Das optische Element 220 ist dazu eingerichtet, Licht zumindest eines Teilbereichs des ersten Spektralbereichs 214 abzuschwächen und Licht zumindest eines Teilbereichs des zweiten Spektralbereichs 218 durchzulassen. Die sich gegenseitig beeinflussenden Leuchtelemente, insbesondere die Leuchtelemente 212, 216, müssen nicht zwingend an gegenüberliegenden Seiten angeordnet sein. Gemäß der gezeigten Ausführungsform umfasst Licht zumindest des Teilbereichs des ersten Spektralbereichs 214 Licht zumindest im Wesentlichen des gesamten ersten Spektralbereichs 214. Ebenfalls umfasst Licht zumindest des Teilbereichs des zweiten Spektralbereichs 218 Licht zumindest im Wesentlichen des gesamten zweiten Spektralbereichs 218. Gemäß einigen Ausführungsformen, die nicht gezeigt sind, können der erste Spektralbereich 214 und der zweite Spektralbereich 218 zumindest teilweise übereinstimmen.

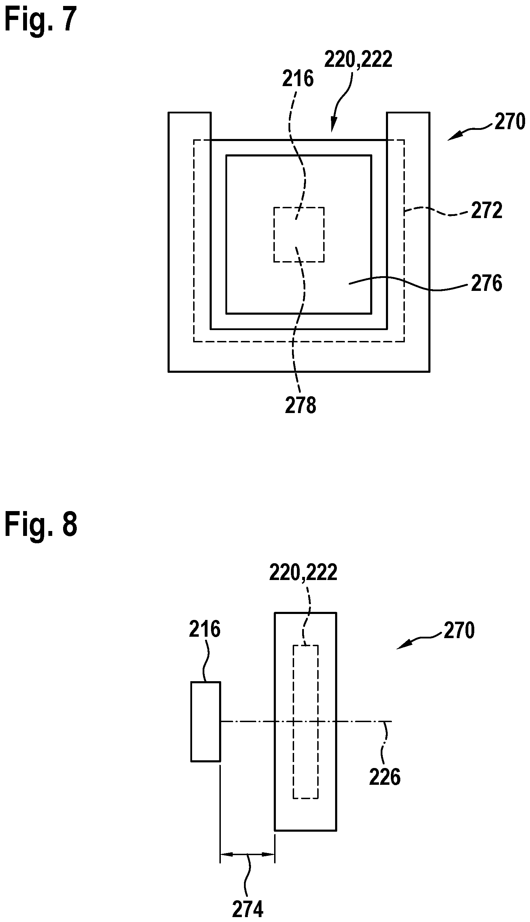

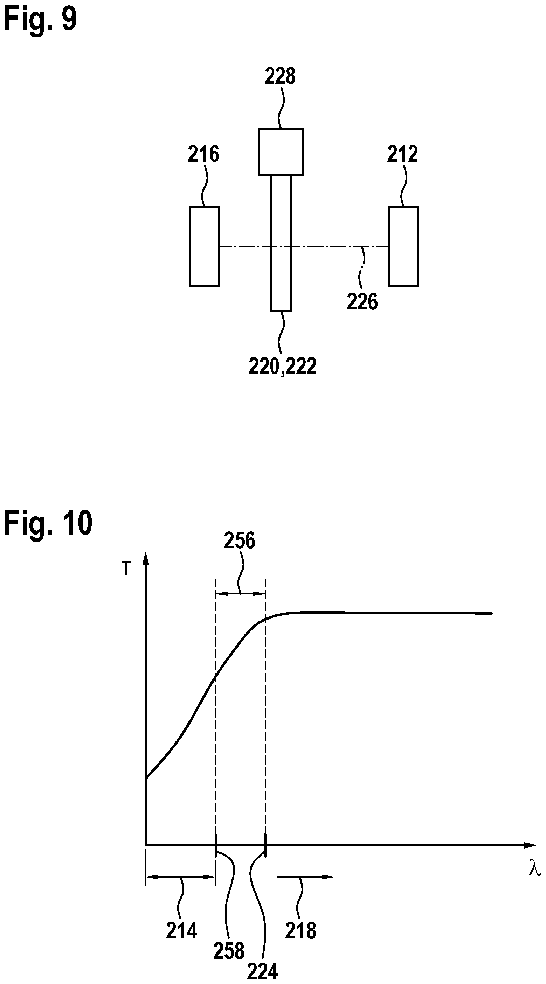

Das optische Element 220 umfasst einen Kantenfilter 222, dessen optische Kante (siehe Fig. 10) bei einer Kantenwellenlänge 224 liegt und der unterhalb der Kantenwellenlänge 224 abschwächend und oberhalb der Kantenwellenlänge 224 durchlassend ausgebildet ist. Der erste Spektralbereich 214 umfasst Licht mit Wellenlängen, die kleiner sind als die Kantenwellenlänge 224, und der zweite Spektralbereich 218 umfasst Licht mit Wellenlängen, die größer sind als die Kantenwellenlänge 224. Das optische Element 220 wird in den Fig. 7 bis 11 genauer beschrieben.

In der Fig. 10 ist eine schematische Darstellung eines Transmissionsdiagramms des Kantenfilters 222 gezeigt. Auf der Ordinate ist eine prozentuale Transmission einfallenden Lichts aufgetragen. Auf der Abszisse ist eine Wellenlänge aufgetragen. Der Kantfilter 222 ist als Hochpassfilter ausgeführt, was bedeutet, dass die Transmission unterhalb der Kantenwellenlänge 224 geringer ist als oberhalb der Kantenwellenlänge 224. Oberhalb der Kantenwellenlänge 224 kann die Transmission zumindest im Wesentlichen 100 % betragen. Ferner kann der zweite Spektralbereich 218 bei der Kantenwellenlänge 224 und/oder bei einer bis zu beispielsweise 20 nm oder bis zu 50 nm höheren Wellenlänge beginnen. Der erste Spektralbereich 214 kann einen oberen Rand 258 aufweisen. Ein Abstand 256 des oberen Rands 258 zu der Kantenwellenlänge 224 kann derart ausgewählt werden, dass die Transmission gering ist, beispielweise maximal 20 %, 10 %, 5 % oder 2 % beträgt. Ferner kann der Abstand 256 derart ausgewählt werden, dass das optische Element 220 gemäß einem Anteil Licht des ersten Spektralbereichs 214 anteilig durchlässt, wobei durch den Abstand 256 der Anteil derart abgestimmt ist, dass in dem Fluoreszenzmodus von dem zweiten Leuchtelement 216 emittiertes Licht, das auf eine Anregung durch Licht des ersten Leuchtelements 212 zurückgeht, als Hintergrundlicht zur Darstellung anatomischer Strukturen verwendbar ist.

Wieder bezugnehmen auf die Fig. 5 sind weitere optische Elemente 230, 238, 240, 242 vor jeweils einem der weiteren Leuchtelemente 232, 234, 236 und dem ersten Leuchtelement 212 angeordnet. Das weitere optische Element 230, das vor dem ersten Leuchtelement 212 angeordnet ist, ist dazu eingerichtet, Licht des ersten Spektralbereichs 214 durchzulassen und Licht abzuschwächen, das bezüglich des ersten Spektralbereichs 212 langwelliger ist. Es ist als ein Tiefpassfilter ausgebildet, der entgegengesetzt analog zu dem optischen Element 220 funktioniert. Alle optischen Elemente 220, 230, 238, 240, 242 sind mit dem Emissionsspektrum der ihnen zugeordneten Leuchtelemente 212, 216, 232, 234, 236 abgestimmt. Ferner sind die optischen Elemente 220, 230, 238, 240, 242 mit den optischen Eigenschaften der Strahlkombinierer 244 und insbesondere den optischen Eigenschaften derer Spiegel (siehe Fig. 2) abgestimmt. Beispielsweise kann das Leuchtelement 234 eine Emissionswellenlänge von 770 nm und das Leuchtelement 236 von 940 nm aufweisen. Die entsprechenden optischen Elemente 240, 242 sind analog zu den optischen Elementen 220, 230 ausgebildet. Insbesondere kann das optische Element 242 einen Kantenfilter umfassen, der als Hochpassfilter ausgebildet ist. Grundsätzlich sind die optischen Elemente 238, 240, 242 dazu eingerichtet, Licht des entsprechenden Leuchtelements 232, 234, 236 durchzulassen und Licht abzuschwächen, das bezüglich des Lichts des entsprechenden Leuchtelements 232, 234, 236 kurzwelliger ist. Das mittels der Leuchtelemente 212, 216, 232, 234, 236 emittierte Licht wird mittels der Strahlteiler 244 auf einen gemeinsamen optischen Pfad gelenkt und zu der optischen Schnittstelle 208 geführt.

Zwischen jedem der Leuchtelemente 212, 216, 232, 234, 236 und den entsprechenden optischen Elementen 220, 230, 238, 240, 242 kann optional eine Linse 260 gemäß der Fig. 2 angeordnet sein. Die Linse kann alternativ oder zusätzlich in das entsprechende Leuchtelement 212, 216, 232, 234, 236 integriert sein.

Zumindest eines der optischen Elemente 220, 230, 238, 240, 242 kann derart ausgebildet sein, dass eine Lichtdurchlässigkeit des optischen Elements 220, 230, 238, 240, 242 schaltbar ist. Beispielsweise kann eines der optischen Elemente 220, 230, 238, 240, 242 als LCD-Fenster ausgebildet sein und/oder eines umfassen. Eine Steuereinheit kann mit dem entsprechenden optischen Element 220, 230, 238, 240, 242 verbunden sein (nicht dargestellt) und dieses zur Reduzierung der Lichtdurchlässigkeit mit Spannung versorgen.

Die Fig. 6 zeigt eine schematische Darstellung eines Lichtintensitätsdiagramms der gesamten Beleuchtungseinheit 210. Es umfasst einen Ausschnitt einer kombinierten Emission aller Leuchtelemente der Beleuchtungseinheit 210, im Folgenden Gesamtspektrum genannt. Auf der Ordinate ist eine Lichtintensität von Licht aufgetragen, das beispielsweise als Beleuchtungslicht an der optischen Schnittstelle 208 auskoppelbar ist. Auf der Abszisse ist eine Wellenlänge aufgetragen. Das dargestellte Lichtintensitätsdiagram betrifft eine Situation, bei der der Strahlkombinierer 244 vorgesehen ist, um emittiertes Licht zweier Leuchtelemente zu kombinieren. Die optischen Eigenschaften des Strahlkombinierers 244, insbesondere der Spiegel des Strahlkombinierers 244, können beispielsweise Toleranzen aufweisen und/oder emittiertes Licht in Randbereichen seines Spektralbereichs in einen zunehmend Spektralbereich fallen, in dem der entsprechende Spiegel zumindest teilweise transmittierend ist. Dadurch kann insbesondere emittiertes Licht eines der Leuchtelemente in ein anderes, insbesondere gegenüberliegendes, Leuchtelement fallen. Das gezeigte Diagramm kann einen solchen Fall betreffen. Das erste der Leuchtelemente 212 weist gemäß diesem Beispiel eine Emissionswellenlänge von 770 nm auf und ist dazu eingerichtet, Licht in dem ersten Spektralbereich 214 zu emittieren. Das zweite Leuchtelement 216 ist dazu eingerichtet, Licht in dem zweiten Spektralbereich 218 zu emittieren. Lediglich das erste Leuchtelement 212 wird vorliegend angesteuert. Man erkennt, dass der erste Spektralbereich 214 einen Wellenlängenpeak 260 aufweist, beziehungsweise durch diesen definiert wird. Der Wellenlängenpeak 260 weist ein Maximum auf, das eine Peakhöhe 264 definiert. Das Maximum liegt zumindest im Wesentlichen bei der angegebenen Emissionswellenlänge des ersten Leuchtelements 212. Der Wellenlängenpeak 260 weist ferner eine Breite 266 auf. Der Wellenlängenpeak 260 ragt deutlich aus einer Hintergrundintensität 268 hervor. Der erste Spektralbereich 214 entspricht gemäß der Fig. 6 der Breite 266 des Wellenlängenpeaks 260. Dies ist beispielhaft zu verstehen. Aufgrund der Emission des ersten Leuchtelements 212 von Licht im ersten Spektralbereich 214 wird eine Emission des zweiten Leuchtelements 216 zumindest im Wesentlichen im zweiten Spektralbereich 218 angeregt. Das zweite Leuchtelement 212 ist nicht aktiviert, emittiert aber dennoch Licht. Dies ist der Fall, wenn kein erfindungsgemäßes optisches Element 220 vorgesehen wird. Das Gesamtspektrum weist daher den zweiten Wellenlängenpeak 262 auf, das auf die Lichtemission des zweiten Leuchtelements 216 zurückzuführen ist. Der zweite Wellenlängenpeak 262 kann beispielhaft ein Maximum bei der Emissionswellenlänge des zweiten Leuchtelements 216, vorliegend 940 nm, aufweisen. Das Maximum kann jedoch auch bei einer verschiedenen Wellenlänge liegen, insbesondere innerhalb des zweiten Spektralbereichs 218. Wenn das optische Element 220 vor dem zweiten Leuchtelement 216 angeordnet wird, reduziert sich die Peakhöhe des zweiten Wellenlängenpeaks 262. Dies ist beispielhaft durch die gestrichelten Linien dargestellt. Dies liegt daran, da das Licht im ersten Spektralbereich abgeschwächt wird. Der zweite Wellenlängenpeak 262 kann derart abgeschwächt werden, dass das emittierte Licht des zweiten Leuchtelements 216 als Hintergrundlicht verwendet werden kann. Der Wellenlängenpeak 262 wird also lediglich reduziert (obere gestrichelte Linie). Das optische Element 220 kann auch derart wirken, dass Licht des ersten Spektralbereichs 214 zumindest im Wesentlichen vollständig blockiert wird. Der zweite Wellenlängenpeak 262 ist dann zumindest im Wesentlichen nicht mehr vorhanden (untere gestrichelte Linie).

Fig. 7 und Fig. 8 zeigen eine Halterung 270 für das optische Element 220 in einer schematischen Darstellung. In der Fig. 7 ist eine Ansicht auf das zweite Leuchtelement 216 von dem Strahlteiler (siehe Fig. 5) aus gezeigt. In Fig. 8 ist eine Seitenansicht gezeigt. Das optische Element 220 umfasst den Kantenfilter 222 und weist eine optisch wirkende Scheibe 276 auf, durch die Licht zumindest teilweise hindurchtreten kann. Die Scheibe 276 ist 3 mm dick, aus Glas ausgebildet und weist Beschichtungen auf, die die optischen Eigenschaften beeinflussen. Das optische Element 220 ist um einen Abstand 274 beabstandet vor dem Leuchtelement 216 angeordnet. Der Abstand beträgt beispielsweise 1 cm. Dadurch kann seitlich zumindest im Wesentlichen kein Licht, insbesondere des ersten Leuchtelements 212, an dem Element 220 vorbei auf das Leuchtelement 216 fallen. Dies ist auch dadurch gewährleistet, da die Scheibe 276 größer als eine Emissionsfläche 278 des Leuchtelements 216 ausgebildet ist und diese beidseitig in beide Erstreckungsrichtungen überragt. Insbesondere überragt die Scheibe 276 die Emissionsfläche 278 beidseitig in beiden Erstreckungsrichtungen um mindestens eine Breite und Höhe der Emissionsfläche 278. Das Leuchtelement 216 weist eine optische Achse 226 auf, in der das optische Element 220, insbesondere mittig, angeordnet ist. Das optische Element 220 wird durch die Halterung 270 gehalten. Die Halterung weist eine Aufnahmenut 272 für das optische Element 220 auf. Das optische Element 220 kann in die Aufnahmenut 272 eingeschoben werden. Insbesondere ist das optische Element 220 nicht permanent fixiert angeordnet und kann gegen andere optische Elemente getauscht werden.

Beispielsweise kann zumindest eines der optischen Elemente 230, 238, 240, 242 gegen einen Anregungsfilter getauscht werden. Der Anregungsfilter kann dazu eingerichtet sein, zumindest spektrale Komponenten für ein Beleuchtungslicht für eine Fluoreszenzbildgebung bereitzustellen, wobei der Anregungsfilter durch das entsprechende Leuchtelement angeregt werden kann. Die Anregungsfilter können entsprechend der optischen Elemente 230, 238, 240, 242 anordenbar sein.

Fig. 9 zeigt eine schematische Darstellung eines bewegbar gelagerten optischen Elements 220. Zusätzlich ist das erste Leuchtelement 212 gezeigt, das eine optische Achse 226 aufweist. Die optische Achse 226 fällt mit der optischen Achse 226 des zweiten Leuchtelements zusammen. Daher wird dasselbe Bezugszeichen verwendet. Alternativ können die optischen Achsen auseinanderfallen. Alternativ oder zusätzlich zu der Halterung (Fig. 7) kann ein Antrieb 228 vorgesehen sein, der dazu eingerichtet ist, das optische Element 220 zwischen einer Abschwächungsposition und einer Freigabeposition zu bewegen. Das optische Element 220 ist dazu bewegbar gelagert. In der Abschwächungsposition ist das optische Element 220 in der optischen Achse 226 des ersten Leuchtelements 212 angeordnet. In der Freigabeposition ist das optische Element 220 nicht in der optischen Achse 226 des ersten Leuchtelements 212 angeordnet. Das optische Element 220 kann also wahlweise vorsehbar sein und ein Benutzer beispielsweise den Antrieb 228 steuern. Alternativ oder zusätzlich kann der Antrieb 228 automatisch steuerbar sein, wenn ein Bildgebungsmodus geändert wird. Beispielsweise wenn der Fluoreszenzmodus eingeschaltet wird, kann das optische Element in die Abschwächungsposition bewegt werden, insbesondere automatisch.

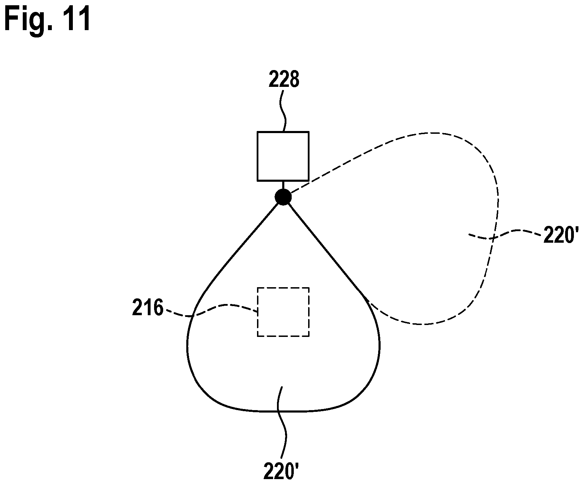

In der Fig. 11 ist eine schematische Darstellung einer weiteren Ausführungsform eines optischen Elements 220` gezeigt. Das optische Element 220` umfasst eine lichtundurchlässige Platte, die mittels des Antriebs 228 vor das Leuchtelement 216 bewegbar ist. Mit der durchgezogenen Linie ist das Element 220' in der Abschwächungsposition dargestellt, in der das optische Element 220' dazu eingerichtet ist, Licht des ersten Spektralbereichs 214 zumindest im Wesentlichen vollständig zu blockieren. Mit der gestrichelten Linie ist das Element 220' in der Freigabeposition dargestellt. Gemäß Fig. 11 ist das optische Element 220` drehbar bewegbar gelagert. Alternativ kann das optische Element 220' auch seitlich vor das Leuchtelement 216 schiebbar sein und/oder klappbar sein.

### Bezugszeichenliste

- 10: Bildgebungsvorrichtung
- 12: Beleuchtungsvorrichtung
- 13: Beleuchtungseinheit
- 14: Bildgebungsgerät
- 16: optische Schnittstelle
- 20: Leuchtelement
- 22: Leuchtelement
- 24: Leuchtelement
- 26: Leuchtelement
- 28: Leuchtelement
- 30: Strahlkombinierer
- 32: Strahlkombinierer
- 34: Eingangsseite
- 36: Eingangsseite
- 37: Eingangsseite
- 38: Eingangsseite
- 40: Eingangsseite
- 41: Eingangsseite
- 42: Ausgangsseite
- 44: Ausgangsseite
- 54: gemeinsamer optischer Pfad
- 56: lichtemittierende Fläche
- 58: lichtemittierende Fläche
- 60: lichtemittierende Fläche
- 62: lichtemittierende Fläche
- 64: lichtemittierende Fläche
- 74: Anzeigeeinheit
- 78: Linse
- 80: Linse
- 82: Linse
- 84: Linse
- 86: Linse
- 88: Linse
- 90: Spiegel
- 92: Spiegel
- 94: Spiegel
- 96: Spiegel
- 98: Transmissionsspektrum
- 100: Transmissionsspektrum
- 102: Transmissionsspektrum
- 104: Transmissionsspektrum
- 200: Beleuchtungsvorrichtung
- 202: Bildgebungsinstrument
- 204: Endoskop
- 206: Exoskop
- 208: optische Schnittstelle
- 210: Beleuchtungseinheit
- 212: erstes Leuchtelement
- 214: erster Spektralbereich
- 216: zweites Leuchtelement
- 218: zweiter Spektralbereich
- 220: optisches Element
- 222: Kantenfilter
- 224: Kantenwellenlänge
- 226: optische Achse
- 228: Antrieb
- 230: weiteres optisches Element
- 232: weiteres Leuchtelement
- 234: weiteres Leuchtelement
- 236: weiteres Leuchtelement
- 238: weiteres optisches Element
- 240: weiteres optisches Element
- 242: weiteres optisches Element
- 244: Strahlkombinierer
- 246: Eingangsseite
- 248: Eingangsseite
- 250: Ausgangsseite
- 252: System
- 254: Innenraum
- 256: Abstand
- 258: oberer Rand
- 260: Wellenlängenpeak
- 262: zweiter Wellenlängenpeak
- 264: Peakhöhe
- 266: Breite
- 268: Hintergrundintensität
- 270: Halterung
- 272: Aufnahmenut
- 274: Abstand
- 276: Scheibe

## Patentansprüche

1. Beleuchtungsvorrichtung (200) zur Bereitstellung von Beleuchtungslicht für ein Bildgebungsinstrument (202), insbesondere ein Endoskop (204) oder Exoskop (206), umfassend:
eine optische Schnittstelle (208) zur optischen Anbindung eines Bildgebungsinstruments (202); und
eine Beleuchtungseinheit (210), die dazu eingerichtet ist, Beleuchtungslicht an die optische Schnittstelle (208) zu liefern, umfassend:
ein erstes Leuchtelement (212), das dazu eingerichtet ist, Licht in einem ersten Spektralbereich (214) zu emittieren;
ein zweites Leuchtelement (216), das dazu eingerichtet ist, Licht in einem zweiten Spektralbereich (218) zu emittieren, der bezüglich des ersten Spektralbereichs (214) langwelligeres Licht umfasst; und
ein optisches Element (220), das vor dem zweiten Leuchtelement (216) angeordnet ist und das dazu eingerichtet ist, Licht zumindest eines Teilbereichs des ersten Spektralbereichs (214) abzuschwächen und Licht zumindest eines Teilbereichs des zweiten Spektralbereichs (218) durchzulassen.

2. Beleuchtungsvorrichtung (200) nach Anspruch 1,
wobei das optische Element (220) einen Kantenfilter (222) umfasst, dessen optische Kante bei einer Kantenwellenlänge (224) liegt und der unterhalb der Kantenwellenlänge (224) abschwächend und oberhalb der Kantenwellenlänge (224) durchlassend ausgebildet ist, wobei der erste Spektralbereich (214) Licht mit Wellenlängen umfasst, die kleiner sind als die Kantenwellenlänge (224), und wobei der zweite Spektralbereich (218) Licht mit Wellenlängen umfasst, die größer sind als die Kantenwellenlänge (224).

3. Beleuchtungsvorrichtung (200) nach Anspruch 1 oder 2,
wobei das optische Element (220) dazu eingerichtet ist, Licht des ersten Spektralbereichs (214) zumindest im Wesentlichen vollständig zu blockieren.

4. Beleuchtungsvorrichtung (200) nach einem der vorhergehenden Ansprüche,
wobei die Beleuchtungseinheit (210) multimodal ausgebildet und in mehreren unterschiedlichen Beleuchtungsmodi betreibbar ist,
wobei die Beleuchtungsmodi zumindest einen Fluoreszenzmodus umfassen, in dem das erste Leuchtelement (212) aktiviert ist und in dem das zweite Leuchtelement (216) deaktiviert ist, und
wobei die Beleuchtungsmodi zumindest einen weiteren Beleuchtungsmodus umfassen, in dem zumindest das zweite Leuchtelement (216) aktiviert ist.

5. Beleuchtungsvorrichtung (200) nach Anspruch 4,
wobei zumindest eine Komponente des zweiten Leuchtelements (216) durch Licht in dem ersten Spektralbereich (214) derart anregbar ist, dass das zweite Leuchtelement (216) aufgrund der Anregung Licht im zweiten Spektralbereich (218) emittiert; und
wobei das optische Element (220) dazu eingerichtet ist, Licht des ersten Spektralbereichs (214) gemäß einem Anteil anteilig durchzulassen.

6. Beleuchtungsvorrichtung (200) nach Anspruch 5,
wobei der Anteil derart abgestimmt ist, dass in dem Fluoreszenzmodus von dem zweiten Leuchtelement (216) emittiertes Licht, das auf eine Anregung durch Licht des ersten Leuchtelements (212) zurückgeht, als Hintergrundlicht zur Darstellung anatomischer Strukturen verwendbar ist.

7. Beleuchtungsvorrichtung (200) nach einem der vorhergehenden Ansprüche,
wobei das optische Element (220) bewegbar gelagert ist und zwischen einer Abschwächungsposition, in der das optische Element (220) in einer optischen Achse (226) des ersten Leuchtelements (212) angeordnet ist, und eine Freigabeposition, in der optische Element (220) nicht in der optischen Achse (226) des ersten Leuchtelements (212) angeordnet ist, bewegbar ist.

8. Beleuchtungsvorrichtung (200) nach Anspruch 7,
wobei die Beleuchtungseinheit (210) ferner einen Antrieb (228) umfasst, der dazu eingerichtet ist, das optische Element (220) zwischen der Abschwächungsposition und der Freigabeposition zu bewegen.

9. Beleuchtungsvorrichtung (200) nach einem der vorhergehenden Ansprüche,
wobei eine Lichtdurchlässigkeit des optischen Elements (220) schaltbar ist.

10. Beleuchtungsvorrichtung (200) nach einem der vorhergehenden Ansprüche,
wobei die Beleuchtungsvorrichtung (200) ferner ein weiteres optisches Element (230) umfasst, das vor dem ersten Leuchtelement (212) angeordnet ist und das dazu eingerichtet ist, Licht des ersten Spektralbereichs (214) durchzulassen und Licht abzuschwächen, das bezüglich des ersten Spektralbereichs (212) langwelliger ist.

11. Beleuchtungsvorrichtung (200) nach einem der vorhergehenden Ansprüche,
wobei die Beleuchtungsvorrichtung (200) ferner umfasst:
ein oder mehrere weitere/s Leuchtelement/e (232, 234, 236), das/die dazu eingerichtet ist/sind, in unterschiedlichen Spektralbereichen Licht zu emittieren, und
ein oder mehrere weitere optische Elemente (238, 240, 242), wobei vor jedem der Leuchtelemente (232, 234, 236) ein optisches Element (238, 240, 242) angeordnet ist, das dazu eingerichtet ist, Licht des entsprechenden Leuchtelements (232, 234, 236) durchzulassen und Licht abzuschwächen, das bezüglich des Lichts des entsprechenden Leuchtelements (232, 234, 236) kurzwelliger ist.

12. Beleuchtungsvorrichtung (200) nach einem der vorhergehenden Ansprüche,
wobei die Beleuchtungseinheit (210) zumindest einen Strahlkombinierer (244) umfasst, der dazu eingerichtet ist, Licht eines der Leuchtelemente (212, 216, 232, 234, 236) mit Licht eines anderen der Leuchtelemente (212, 216, 232, 234, 236) zu kombinieren und in Richtung der optischen Schnittstelle (208) zu führen.

13. Beleuchtungsvorrichtung (200) nach einem der vorhergehenden Ansprüche,
wobei die Beleuchtungseinheit (210) zumindest einen gekreuzten Strahlkombinierer (244) umfasst, mittels dessen Licht von gegenüberliegenden Eingangsseiten (246, 248) zu einer Ausgangsseite (250) ablenkbar ist, und
wobei auf den gegenüberliegenden Eingangsseiten (246, 248) des gekreuzten Strahlkombinierers (244) jeweils zumindest eines der Leuchtelemente (212, 216, 232, 234, 236) angeordnet ist.

14. System (252), insbesondere optisches System, umfassend:
eine Beleuchtungsvorrichtung (200) nach einem der vorhergehenden Ansprüche; und
ein Bildgebungsinstrument (202), insbesondere ein Endoskop (204) oder Exoskop (206), das an die optische Schnittstelle (208) der Beleuchtungsvorrichtung (200) anbindbar und/oder angebunden ist.
